# EUROPEAN PATENT APPLICATION

(11) **EP 4 620 923 A1**
(43) Date of publication of application: **24.09.2025**
(21) Application number: 25155273.3
(22) Date of filing: 31.01.2025
(51) Int. Cl.: C02F 1/44, H01L 21/00

(54) **ORGANIC SOLVENT COLLECTION APPARATUS, SUBSTRATE PROCESSING APPARATUS, AND ORGANIC SOLVENT COLLECTION METHOD**

(30) Priority: 19.03.2024 JP 2024043314
(71) Applicant: SCREEN Holdings Co., Ltd., Kyoto-shi, Kyoto 602-8585 (JP)
(72) Inventor: IWAO, Michinori, Kyoto, 602-8585 (JP); YOSHIDA, Yukifumi, Kyoto, 602-8585 (JP); UEMURA, Tomohiro, Kyoto, 602-8585 (JP); MINAMI, Shoyo, Kyoto, 602-8585 (JP); UEDA, Yusuke, Kyoto, 602-8585 (JP)
(74) Representative: Kilian Kilian & Partner mbB

(57) **Abstract**

An organic solvent collection apparatus includes at least one collection tank, a dewatering circulator, and a purification circulator. The collection tank stores a mixed liquid of an organic solvent and water. The dewatering circulator includes at least one dewatering circulation pipe connected to the collection tank and a circulation dewaterer that separates water from the mixed liquid, and generates a first concentrated liquid that is a mixed liquid in which a solvent concentration of the organic solvent is increased by circulation through the dewatering circulation pipe. The purification circulator includes at least one purification circulation pipe through which the first concentrated liquid is circulated and at least one filter that captures an impurity of the first concentrated liquid, and circulates the first concentrated liquid through the purification circulation pipe to generate a recycling liquid in which a content rate of the impurity in the first concentrated liquid is reduced.

## Description

### BACKGROUND

### Technical Field

The present disclosure relates to an organic solvent collection apparatus, a substrate processing apparatus, and an organic solvent collection method.

### Description of the Background Art

Japanese Patent Application Laid-Open No. 2017-41505 discloses an IPA collection system that collects water-containing IPA (isopropyl alcohol) discharged from a processing unit processing a substrate. The IPA collection system includes a storage tank, a circulation pipe, a pump, a dewatering unit, and a filter. Water-containing IPA from the processing unit is supplied to the storage tank. The circulation pipe is connected to the storage tank, and returns the water-containing IPA from the storage tank to the storage tank. The pump is provided in the circulation pipe, and sends the water-containing IPA from an upstream end toward a downstream end of the circulation pipe. The filter is provided in the circulation pipe to remove foreign substances in the water-containing IPA. The dewatering unit is provided in the circulation pipe to remove moisture from the water-containing IPA.

The IPA collection system circulates the water-containing IPA through a circulation path including the storage tank and the circulation pipe. This circulation causes the water-containing IPA to pass through the filter and the dewatering unit. Thus, an IPA concentration of the water-containing IPA during the circulation increases, and the foreign matters in the water-containing IPA are reduced. That is, by this circulation, the clean water-containing IPA having the high IPA concentration is stored in the storage tank. The water-containing IPA in this storage tank is again supplied to the processing unit. Thus, an amount of discarded IPA can be reduced.

### SUMMARY

When the dewatering unit and the filter are provided on the same circulation path, it is difficult to circulate the water-containing IPA under a condition suitable for both the dewatering unit and the filter (for example, pressure, flow rate or temperature). For this reason, it has been difficult to achieve both efficient improvement of a solvent concentration by removing moisture in the dewatering unit and efficient cleaning by the filter.

The present disclosure is directed to an organic solvent collection apparatus, a substrate processing apparatus, and an organic solvent collection method.

One aspect of the present disclosure is an organic solvent collection apparatus includes: at least one collection tank that stores a mixed liquid of an organic solvent and water discharged from a processing unit that processes a substrate; a dewatering circulator that includes at least one dewatering circulation pipe connected to the at least one collection tank and a circulation dewaterer provided in the at least one dewatering circulation pipe and separating water from the mixed liquid, the dewatering circulator generating a first concentrated liquid that is the mixed liquid in which a solvent concentration of the organic solvent is increased by circulation through the at least one dewatering circulation pipe; and a purification circulator that includes at least one purification circulation pipe circulating the first concentrated liquid and at least one filter provided in the at least one purification circulation pipe and capturing an impurity of the first concentrated liquid, the purification circulator recycling the first concentrated liquid through the at least one purification circulation pipe to generate a recycling liquid that is the first concentrated liquid in which a content rate of the impurity is reduced.

The mixed liquid can be circulated through the dewatering circulation pipe under a condition corresponding to the circulation dewaterer, and the first concentrated liquid can be circulated through the purification circulation pipe under a condition corresponding to the filter.

Preferably, the circulation dewaterer includes a membrane separator having a separation membrane that allows water to pass therethrough.

Thus, the apparatus scale can be reduced as compared with the distiller.

Preferably, the circulation dewaterer includes a dewatering temperature regulator that heats the mixed solution.

This makes it possible to efficiently separate the water from the mixed liquid.

Preferably, the purification circulator further includes a purification temperature regulator that heats or cools the first concentrated solution flowing through the at least one purification circulation pipe.

Thus, the temperature of the mixed solution can be adjusted to the temperature suitable for the filter.

Preferably, the purification temperature regulator cools the first concentrated solution flowing through the at least one purification circulation pipe.

Thus, even when the purification temperature regulator increases the temperature of the mixed liquid, the high-temperature first concentrated liquid can be cooled to near the normal temperature. For this reason, thermal deformation of the filter can be prevented, and the filter can capture the impurities with high efficiency.

Preferably, the organic solvent collection apparatus further includes: at least one purification tank; and a first liquid sending pipe that causes the first concentrated liquid from the at least one collection tank to flow toward the at least one purification tank, in which the at least one purification circulation pipe returns the first concentrated liquid from the at least one purification tank to the at least one purification tank.

Thus, when the first concentrated liquid having the high temperature is supplied to the purification tank through the first liquid sending pipe, the temperature of the first concentrated liquid can be lowered by heat dissipation during passage through the first liquid sending pipe.

Preferably, the organic solvent collection apparatus further includes a collection selector that selects a collection destination of the mixed liquid from the processing unit between a first collection tank and a second collection tank included in said at least one collection tank, in which the at least one dewatering circulation pipe includes a first dewatering circulation pipe and a second dewatering circulation pipe, the first dewatering circulation pipe returns the mixed liquid from the first collection tank to the first collection tank, and the second dewatering circulation pipe returns the mixed liquid from the second collection tank to the second collection tank.

Thus, the mixed liquid from the processing unit can be collected in the second collection tank during the circulation of the mixed liquid through the first dewatering circulation pipe, and the mixed liquid from the processing unit can be collected in the first collection tank during the circulation of the mixed liquid through the second dewatering circulation pipe. In short, the mixed liquid from the processing unit can be collected even during the circulation of the mixed liquid through the first dewatering circulation pipe or during the circulation of the mixed liquid through the second dewatering circulation pipe. Conversely, the mixed liquid having the low solvent concentration discharged from the processing unit can be prevented from flowing into the mixed liquid during the circulation. For this reason, the drop in the solvent concentration of the mixed liquid in the circulation can be prevented.

Preferably, the first dewatering circulation pipe includes a first upstream pipe having an upstream end connected to the first collection tank, a first downstream pipe having a downstream end connected to the first collection tank, and a common circulation pipe connected between the first upstream pipe and the first downstream pipe, the second dewatering circulation pipe includes a second upstream pipe having an upstream end connected to the second collection tank, a second downstream pipe having a downstream end connected to the second collection tank, and the common circulation pipe connected between the second upstream pipe and the second downstream pipe, the circulation dewaterer is provided in the common circulation pipe, and the dewatering circulator further includes a circulation selecting valve part that selects between circulation through the first dewatering circulation pipe and circulation through the second dewatering circulation pipe.

Thus, the circulation dewaterer is commonly used in the first dewatering circulation pipe and the second dewatering circulation pipe, so that the apparatus scale and the manufacturing cost can be reduced.

Preferably, the organic solvent collection apparatus further includes a concentrated liquid supply part that selects a supply destination from the at least one collection tank between a first purification tank and a second purification tank included in the at least one purification tank, in which the at least one purification circulation pipe includes a first purification circulation pipe and a second purification circulation pipe, the first purification circulation pipe returns the first concentrated liquid from the first purification tank to the first purification tank, the second purification circulation pipe returns the first concentrated liquid from the second purification tank to the second purification tank, the at least one filter includes a first filter and a second filter, the first filter is provided in the first purification circulation pipe, and the second filter is provided in the second purification circulation pipe.

Thus, the circulation through the first filter and the circulation through the second filter can be performed in parallel. For this reason, the recycling liquid having the lower impurity content rate can be generated.

Preferably, the organic solvent collection apparatus includes: a separation discharge pipe through which a separation fluid containing water separated from the mixed liquid by the circulation dewaterer as a main component flows; and a decomposer that is provided in the separation discharge pipe and decomposes the organic solvent contained in the separation fluid.

Thus, the liquid discharge process of the separation fluid at the discharge destination can be simplified.

Preferably, the organic solvent collection apparatus further includes a condenser that is provided in the separation discharge pipe on an upstream side of the decomposer and condenses vapor of the organic solvent contained in the separation fluid, in which the decomposer electrolyzes the organic solvent of liquid.

Thus, the organic solvent is electrolyzed after the vapor of the organic solvent is condensed, so that the organic solvent can be more reliably decomposed.

Preferably, the organic solvent collection apparatus further includes: a separation discharge pipe through which a separation fluid containing water as a main component separated from the mixed liquid by the circulation dewaterer flows; a separation dewaterer that is provided in the separation discharge pipe and separates the organic solvent from the separation fluid; and a return pipe that is connected to the separation dewaterer and joins a second concentrated liquid containing the organic solvent separated from the separation fluid by the separation dewaterer in the first concentrated liquid.

Thus, the second concentrated liquid containing the organic solvent in the separation fluid separated by the circulation dewaterer joins in the first concentrated liquid. For this reason, the discharge amount of the organic solvent can be further reduced.

Preferably, the separation dewaterer includes: at least one adsorption filter that is provided in the separation discharge pipe and adsorbs the organic solvent; at least one heater that heats the at least one adsorption filter to release the organic solvent from the at least one adsorption filter and causes the organic solvent to flow out to the return pipe; and a condenser provided in the return pipe.

Thus, the organic solvent can be more reliably separated from the separation fluid having the low solvent concentration.

Preferably, the organic solvent collection apparatus includes: a dewatering selector that selects the at least one adsorption filter through which the separation fluid flows between a first adsorption filter and a second adsorption filter in included the at least adsorption filter; and a release selector that selects the adsorption filter communicating with the condenser between the first adsorption filter and the second adsorption filter, in which the at least one heater includes a first heater and a second heater, the first heater heats the first adsorption filter, the second heater heats the second adsorption filter.

Thus, the release by one of the first and second adsorption filters can be performed in parallel with the adsorption of the other of the first and second adsorption filters.

Another aspect of the present disclosure is a substrate processing apparatus, and the substrate processing apparatus includes: the organic solvent collection apparatus; the processing unit; a supply tank to which the recycling liquid is supplied and that stores the recycling liquid; and a second liquid sending pipe that connects the supply tank and the processing unit.

Still another aspect of the present disclosure is an organic solvent collection method includes: a dewatering circulation step of circulating a mixed liquid of an organic solvent and water through a dewatering circulation path including a circulation dewaterer that separates water from the mixed liquid to generate a first concentrated liquid in which a solvent concentration of the organic solvent in the mixed liquid is increased; and a purification circulation step of circulating the first concentrated liquid through a purification circulation path including a filter that captures an impurity in the first concentrated liquid to generate a recycling liquid in which a content rate of the impurity in the first concentrated liquid is reduced.

Preferably, the mixed solution is heated in the dewatering circulation step, and the first concentrated solution is cooled in the purification circulation step.

Thus, the high-temperature first concentrated liquid can be cooled to near the normal temperature. For this reason, thermal deformation of the filter can be prevented, and the filter can capture the impurities with high efficiency.

Therefore, an object of the present disclosure is to provide a technique capable of circulating the mixed liquid of the organic solvent and the water under conditions suitable for each of the dewaterer and the filter.

### BRIEF DESCRIPTION OF THE DRAWINGS

FIG. 1 is a plan view schematically illustrating an example of a substrate processing apparatus;
FIG. 2 is a side view schematically illustrating an example of a processing unit;
FIG. 3 is a view schematically illustrating an example of an organic solvent collection part according to a first embodiment;
FIG. 4 is a flowchart illustrating an example of an operation of the organic solvent collection part;
FIG. 5 is a view schematically illustrating an example of an organic solvent collection part according to a second embodiment;
FIG. 6 is a view schematically illustrating an example of the operation of the organic solvent collection part;
FIG. 7 is a view illustrating an operation example of the organic solvent collection part;
FIG. 8 is a view illustrating an operation example of the organic solvent collection part;
FIG. 9 is a view illustrating an operation example of the organic solvent collection part;
FIG. 10 is a view illustrating an operation example of the organic solvent collection part;
FIG. 11 is a view illustrating an operation example of the organic solvent collection part;
FIG. 12 is a view illustrating an operation example of the organic solvent collection part;
FIG. 13 is a view illustrating an operation example of the organic solvent collection part;
FIG. 14 is a view illustrating an operation example of the organic solvent collection part;
FIG. 15 is a view schematically illustrating an example of an organic solvent collection part according to a third embodiment;
FIG. 16 is a view schematically illustrating a first example of an organic solvent collection part according to a fourth embodiment;
FIG. 17 is a view schematically illustrating a second example of the organic solvent collection part according to the fourth embodiment;
FIG. 18 is a view schematically illustrating an example of the operation of the organic solvent collection part according to the second example of the fourth embodiment;
FIG. 19 is a view illustrating an operation example of the organic solvent collection part;
FIG. 20 is a view illustrating an operation example of the organic solvent collection part;
FIG. 21 is a view illustrating an operation example of the organic solvent collection part; and
FIG. 22 is a view illustrating an operation example of the organic solvent collection part.

### DESCRIPTION OF THE PREFERRED EMBODIMENTS

Hereinafter, embodiments will be described in detail with reference to the drawings. In the drawings, dimensions and numbers of each part are exaggerated or simplified as necessary for easy understanding. Portions having similar configurations and functions are denoted by the same reference numeral, and redundant description will be omitted in the following description.

In the following description, the same components are denoted by the same reference numeral, and it is assumed that names and functions of the same components are also similar. Consequently, the detailed description of the same component is occasionally omitted in order to avoid duplication.

In the following description, even when ordinal numbers such as "first" or "second" are used, these terms are used only for convenience to facilitate understanding of contents of the embodiments, and are not limited to the order that can be generated by these ordinal numbers.

In the case where expressions indicating a relative or absolute positional relationship (for example, "in one direction", "along one direction", "parallel", "orthogonal", "center", "concentric", and "coaxial") are used, the expressions shall not only strictly represent a positional relationship, but also represent a state of being displaced relative to an angle or a distance to an extent that a tolerance or a comparable function is obtained, unless otherwise specified. When expressions indicating an equal state (for example, "same", "equal", and "homogeneous") are used, unless otherwise specified, the expressions shall not only represent a quantitatively strictly equal state, but also represent a state in which there is a difference in obtaining a tolerance or a similar function. In the case where expressions indicating a shape (for example, "quadrangular" or "cylindrical") are used, unless otherwise specified, the expressions shall not only represent the shape geometrically and strictly, but also represent a shape having, for example, unevenness or chamfering within a range in which the same level of effect can be obtained. When expressions "comprising", "owning", "possessing", "including" or "having" one component are used, the expressions are not an exclusive expression excluding presence of other components. When the expression "at least any one of A, B, and C" is used, the expression includes only A, only B, only C, any two of A, B and C, and all of A, B and C.

### <First embodiment>

### <1. substrate processing apparatus>

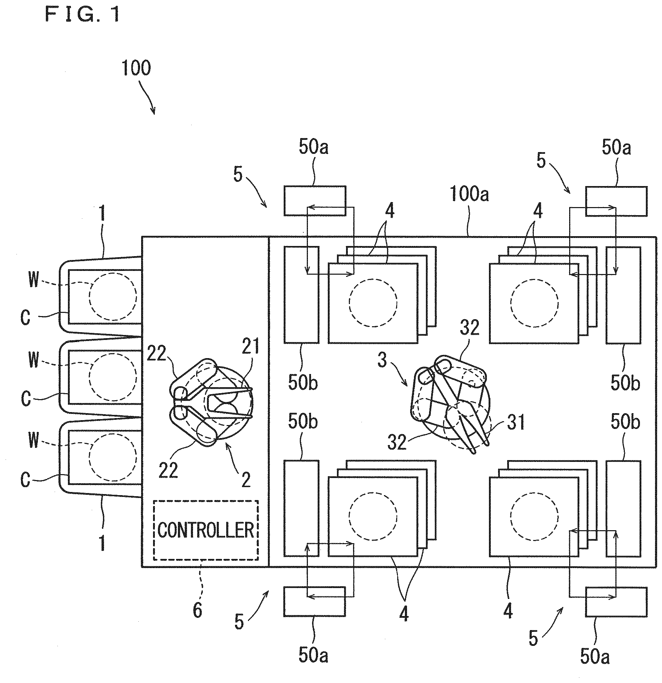

A substrate processing apparatus 100 according to an embodiment will be described with reference to FIG. 1. FIG. 1 is a plan view schematically illustrating an example of the substrate processing apparatus 100.

The substrate processing apparatus 100 is a single wafer type processing apparatus that processes a substrates W, which is a processing target, one by one. For example, the substrate W that is the processing target processed by the substrate processing apparatus 100 is a semiconductor substrate. For example, the shape of the substrate W that is the processing target is a disk shape.

The substrate processing apparatus 100 includes a load port 1, an indexer robot 2, a main conveyance robot 3, a processing unit 4, an organic solvent collection part 5, and a controller 6.

The load port 1 is an interface taking in and out the substrate W with respect to a carrier C that is a kind of storage container storing a plurality of substrates. For example, a plurality of (three in the example of the drawing) load ports 1 are provided. For example, the plurality of load ports 1 are arrayed in a line in a horizontal direction. The carrier C may be of a type in which the substrate W is stored in a sealed space (for example, a front opening unified pod (FOUP), a standard mechanical interface (SMIF) pod, or the like), or may be of a type in which the substrate W is exposed to outside air (for example, an open cassette (OC)).

The indexer robot 2 is a conveyance apparatus that conveys the substrate W. As an example, the indexer robot 2 is a horizontal articulated robot, and includes a pair of hands 21, 21 holding the substrate W and an arm 22 connected to each hand 21. In addition, the indexer robot 2 includes a drive mechanism (not illustrated) that turns each hand 21 and bends and stretches, turns, and lifts and lowers each arm 22. The indexer robot 2 conveys the substrate W between the carrier C held in the load port 1 and the main conveyance robot 3. That is, the indexer robot 2 accesses the carrier C placed in the load port 1 and performs a carry-out operation (that is, the operation of taking out the substrate W stored in the carrier C with the hand 21) and a carry-in operation (that is, the operation of accommodating the substrate W held by the hand 21 in the carrier C). Furthermore, the indexer robot 2 accesses a transfer position and transfers the substrate W to and from the main conveyance robot 3.

The main conveyance robot 3 is a conveyance apparatus that conveys the substrate W. As an example, the main conveyance robot 3 is a horizontal articulated robot, and includes a pair of hands 31, 31 holding the substrate W and an arm 32 connected to each hand 31. In addition, the main conveyance robot 3 includes a drive mechanism (not illustrated) that turns each hand 31 and bends and stretches, turns, and lifts and lowers each arm 32. The main conveyance robot 3 conveys the substrate W between the indexer robot 2 and each processing unit 4. That is, the main conveyance robot 3 accesses the transfer position and transfers the substrate W to and from the indexer robot 2. The main conveyance robot 3 accesses the processing unit 4 and performs the carry-in operation (that is, an operation of carrying the substrate W held by the hand 31 in the processing unit 4) and the carry-out operation (that is, the operation of carrying out the substrate W in the processing unit 4 with the hand 31).

The processing unit 4 performs predetermined processing on the substrate W using a processing liquid (for example, a chemical liquid, a rinse liquid, and IPA). At this point, for example, a plurality of (for example, three) processing units 4 stacked in a vertical direction configure one tower, and the plurality of (for example, four in the example of the drawing) towers are provided so as to surround the main conveyance robot

### 3. A specific configuration of the processing unit 4 will be described later.

The organic solvent collection part 5 collects an organic solvent from the processing unit 4, purifies the collected organic solvent, and supplies the organic solvent to the processing unit 4 again. As an example, the organic solvent collection part 5 may be provided in one-to-one correspondence with each of the plurality of towers, and each organic solvent collection part 5 may collect and supply the organic solvent to each processing unit 4 included in the corresponding tower. A specific configuration of the organic solvent collection part 5 will be described later.

The controller 6 controls the operation of each unit (the load port 1, the indexer robot 2, the main conveyance robot 3, the processing unit 4, and the organic solvent collection part 5) included in the substrate processing apparatus 100. For example, the controller 6 is configured by a general computer having an electric circuit. As an example, the controller 6 includes a central processor unit (CPU) as a central processing unit that performs various types of arithmetic processing (data processing), a read only memory (ROM) that stores a basic program and the like, a random access memory (RAM) that is used as a work area when the CPU performs predetermined processing (data processing), a storage device configured by a nonvolatile storage device such as a flash memory or a hard disk device, a bus line that connects these, and the like. A program that defines processing executed by the controller 6 may be stored in the storage device, the RAM, or the like. In this case, for example, when the CPU executes the program, each part of the substrate processing apparatus 100 may be controlled by the controller 6, and the processing defined by the program may be executed in the substrate processing apparatus 100. That is, when the CPU executes the program, a circuit that performs the processing defined by the program may be implemented by the controller 6. However, a part or all of the control performed by the controller 6 (a part or all of the circuit implemented by the controller 6) may be executed (implemented) by hardware such as a dedicated logic circuit.

### <2. Processing unit>

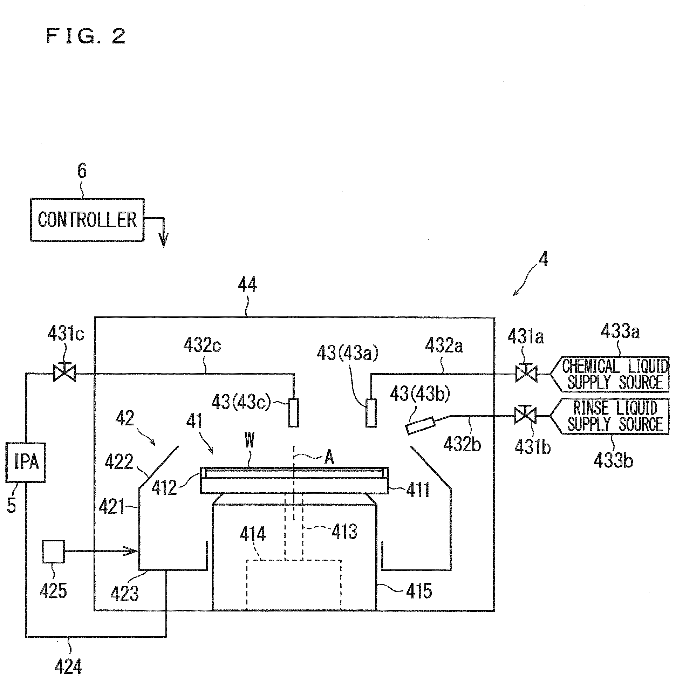

The processing unit 4 will be described with reference to FIG. 2. FIG. 2 is a side view schematically illustrating an example of the processing unit 4.

### <2-1. Configuration of processing unit>

The processing unit 4 performs predetermined processing on the substrate W using a processing liquid (for example, a chemical liquid, a rinse liquid, and IPA). For example, the processing unit 4 includes a spin chuck 41, a cup 42, and a nozzle 43. The spin chuck 41, the cup 42, and the nozzle 43 are accommodated in the processing chamber 44.

The spin chuck 41 rotates the substrate W about an axis (rotational axis) A extending vertically through a center of a main surface while holding the substrate W in a horizontal posture (a posture in which a thickness direction of the substrate W is along an up and down direction (vertical direction)). Specifically, for example, the spin chuck 41 includes a spin base 411. The spin base 411 is a disk-shaped member, and is disposed in a posture in which the thickness direction is along the up and down direction. A plurality of chuck pins 412 are provided on an upper surface of the spin base 411. The plurality of chuck pins 412 is disposed at equal intervals along a circumference corresponding to a peripheral edge of the substrate W. A link mechanism (not illustrated) that moves the chuck pins 412 between an abutting position and an open position is connected to the plurality of chuck pins 412. The "abutting position" is a position at which the chuck pin 412 abuts on the peripheral edge of the substrate W. The "open position" is a position where the chuck pin 412 is away from the peripheral edge of the substrate W. When each of the plurality of chuck pins 412 is disposed at the abutting position, the substrate W is held (chucked) above the spin base 411 in a horizontal posture. When each of the plurality of chuck pins 412 is disposed at the open position, the holding of the substrate W is released. The link mechanism selects the position of the chuck pin 412 according to an instruction from the controller 6. That is, timing of holding the substrate W, timing of releasing the holding of the substrate W, and the like are controlled by the controller 6. In addition, the spin base 411 is connected to a spin motor 414 through a shaft part 413 provided coaxially with the rotational axis A. The shaft part 413 and the spin motor 414 are accommodated in a cover 415. The spin motor 414 rotates the shaft part 413 around the rotational axis A. Thus, the spin base 411 and thus the substrate W held above the spin base 411 rotate around the rotational axis A. The spin motor 414 rotates the spin base 411 according to the instruction from the controller 6. That is, a rotation number of the substrate W, rotation start timing, rotation end timing, and the like of the spin base 411 (and thus the substrate W) are controlled by the controller 6.

The cup 42 catches the processing liquid discharged from the substrate W held and rotated by the spin chuck 41. Specifically, for example, the cup 42 includes a cylindrical guide part 421 disposed coaxially with the rotational axis A, an inclined part 422 that is continuous with an upper end of the guide part 421 and reduces in diameter upward, and a liquid receiver 423 that is continuous with a lower end of the guide part 421 and forms an annular groove opened upward. A cup-side collection pipe (specifically, for example, a cup-side collection pipe (not illustrated) for a chemical solution and a cup-side collection pipe 424 for IPA are used) that collects the liquid caught by the liquid receiver 423 are provided in the liquid receiver 423. In addition, a cup lifting mechanism 425 that lifts and lowers the cup 42 between a lower position and an upper position is connected to the cup 42. The "lower position" is a position where the upper end (specifically, the upper end of the inclined part 422) of the cup 42 is disposed below the substrate W held by the spin chuck 41. The "upper position" is a position where the upper end of the cup 42 is disposed above the substrate W held by the spin chuck 41. The cup lifting mechanism 425 moves up and down the cup 42 according to the instruction from the controller 6. That is, the position of the cup 42 is controlled by the controller 6.

The nozzle 43 discharges the processing liquid toward the upper surface of the substrate W held by the spin chuck 41. At this point, for example, the individual nozzle 43 is provided for each type of processing liquid. That is, the nozzle 43 that discharges a chemical solution (hereinafter, also referred to as a "chemical liquid nozzle 43a"), the nozzle 43 that discharges the rinse liquid (hereinafter, also referred to as a "rinse liquid nozzle 43b"), and the nozzle 43 that discharges the IPA (hereinafter, also referred to as an "IPA nozzle 43c") are provided.

The chemical liquid nozzle 43a discharges the chemical liquid toward the upper surface of the substrate W held by the spin chuck 41. The chemical liquid nozzle 43a is connected to a chemical liquid supply source 433a through a chemical liquid pipe 432a in which a chemical liquid valve 431a is inserted. When the chemical liquid valve 431a is opened, the chemical liquid is supplied to the chemical liquid nozzle 43a through the chemical liquid pipe 432a, and the chemical liquid is discharged from the chemical liquid nozzle 43a. The chemical liquid valve 431a is opened and closed according to the instruction from the controller 6. That is, the discharge timing of the chemical liquid from the chemical liquid nozzle 43a is controlled by the controller 6. For example, the chemical liquid is hydrofluoric acid. However, the chemical liquid is not limited to the hydrofluoric acid, but may be a liquid containing at least one of sulfuric acid, acetic acid, nitric acid, hydrochloric acid, hydrofluoric acid, phosphoric acid, ammonia water, hydrogen peroxide water, an organic acid (for example, citric acid or oxalic acid), an organic alkali (for example, tetramethylammonium hydroxide (TMAH)), a surfactant, and a corrosion inhibitor.

The rinse liquid nozzle 43b discharges the rinse liquid toward the upper surface of the substrate W held by the spin chuck 41. The rinse liquid nozzle 43b is connected to the rinse liquid supply source 433b through the rinse liquid pipe 432b in which the rinse liquid valve 431b is inserted. When the rinse liquid valve 431b is opened, the rinse liquid is supplied to the rinse liquid nozzle 432b through the rinse liquid pipe 432b, and the rinse liquid is discharged from the rinse liquid nozzle 43b. The rinse liquid valve 431b is opened and closed according to the instruction from the controller 6. That is, the discharge timing of the rinse liquid from the rinse liquid nozzle 43b is controlled by the controller 6. For example, the rinse liquid is pure water (deionized water). However, the rinse liquid is not limited to the pure water, but may be any of carbonated water, electrolyzed ion water, hydrogen water, ozone water, and hydrochloric acid water having a dilution concentration (for example, about 10 ppm to about 100 ppm).

The IPA nozzle 43c discharges IPA (that is, a liquid containing the IPA as a main component) toward the upper surface of the substrate W held by the spin chuck 41. The IPA nozzle 43c is connected to the organic solvent collection part 5 through the IPA pipe 432c in which the IPA valve 431c is inserted. When the IPA valve 431c is opened, the IPA is supplied to the IPA nozzle 432c through the IPA pipe 432c, and the IPA is discharged from the IPA nozzle 43c. The IPA valve 431c is opened and closed according to the instruction from the controller 6. That is, the discharge timing of the IPA from the IPA nozzle 43c is controlled by the controller 6.

A nozzle moving mechanism that moves the chemical liquid nozzle 43a, the rinse liquid nozzle 43b, and the IPA nozzle 43c between a processing position and a retracted position may be connected to at least one of the chemical liquid nozzle 43a, the rinse liquid nozzle 43b, and the IPA nozzle 43c. The "processing position" is a position where the processing liquid discharged from the nozzles 43a, 43b, 43c is supplied to the substrate W held by the spin chuck 41. The "retracted position" is a position where the nozzles 43a, 43b, 43c are located outside (radially outside) the peripheral edge of the substrate W held by the spin chuck 41 when viewed from above. In this case, the nozzle moving mechanism moves the nozzles 43a, 43b, 43c according to the instruction from the controller 6. That is, the positions of the nozzles 43a, 43b, 43c are controlled by the controller 6.

### <2-2. Operation of processing unit>

An example of the operation of the processing unit 4 will be described. The operation performed by the processing unit 4 is performed under the control of the controller 6 (that is, the controller 6 controls the chuck pin 412, the spin motor 414, the cup lifting mechanism 425, the chemical liquid valve 431a, the rinse liquid valve 431b, the IPA valve 431c, and the like).

When the substrate W is carried in the processing chamber 44 by the main conveyance robot 3, the spin chuck 41 holds the substrate W. Subsequently, the spin chuck 41 starts the rotation.

In this state, the chemical liquid valve 431a is open. Then, the chemical liquid is discharged from the chemical liquid nozzle 43a toward the upper surface of the substrate W held and rotated by the spin chuck 41. Thus, the chemical liquid is supplied to the entire upper surface of the substrate W, and the substrate W is processed by the chemical liquid (chemical liquid processing step). For example, when the hydrofluoric acid is used as the chemical solution, foreign substances such as particles are removed from the substrate W. The cup 42 is disposed at the upper position during the chemical liquid processing step. Consequently, the chemical liquid scattered around the substrate W is caught by the cup 42. That is, the chemical liquid scattered around the substrate W is caught by the inclined part 422, guided downward by the guide part 421, and collected in the liquid receiver 423. The chemical liquid (that is, the chemical liquid collected in the liquid receiver 423) caught by the cup 42 is collected through the cup-side collection pipe (not illustrated) for the chemical liquid.

The chemical liquid valve 431a is closed at a time point when a predetermined time elapses from the start of the discharge of the chemical solution. Then, the discharge of the chemical liquid from the chemical liquid nozzle 43a is stopped. Subsequently, the rinse liquid valve 431b is open. Then, the rinse liquid is discharged from the rinse liquid nozzle 43b toward the upper surface of the substrate W held and rotated by the spin chuck 41. Thus, the rinse liquid is supplied to the entire upper surface of the substrate W, and the chemical liquid adhering to the substrate W is washed away by the rinse liquid (rinsing step). The cup 42 is also disposed at the upper position during the rinsing step. Consequently, the chemical liquid and the rinse liquid that are scattered around the substrate W are caught by the cup 42. The chemical liquid and the rinse liquid that are caught by the cup 42 are collected through the cup-side collection pipe (not illustrated) for the chemical liquid.

The rinse liquid valve 431b is closed at the time point when the predetermined time elapses from the start of the discharge of the rinse liquid. Then, the discharge of the rinse liquid from the rinse liquid nozzle 43b is stopped. Subsequently, the IPA valve 431c is open. Then, the IPA is discharged from the IPA nozzle 43c toward the upper surface of the substrate W held and rotated by the spin chuck 41. Thus, the IPA is supplied to the entire upper surface of the substrate W, and the rinse liquid attached to the substrate W is replaced with the IPA (IPA supply step). The cup 42 is also disposed at the upper position during the IPA supply step. Consequently, the rinse liquid and the IPA that are scattered around the substrate W are caught by the cup 42. The rinse liquid and IPA that are caught by the cup 42 are collected through the cup-side collection pipe 424 for IPA.

The IPA valve 431c is closed at the time point when the predetermined time elapses from the start of the supply of IPA. Then, the discharge of the IPA from the IPA nozzle 43c is stopped. At this stage, the rinse liquid on the substrate W is completely replaced with the IPA, and a liquid film of the IPA covering the entire upper surface of the substrate W is formed. Subsequently, the spin chuck 41 starts high-speed rotation. Thus, the substrate W is rotated at a high speed, and the IPA on the substrate W is shaken off around the substrate W by centrifugal force (spin dry step). The cup 42 is also disposed at the upper position while the substrate W is rotated at the high speed. Consequently, the IPA scattered around the substrate W is caught by the cup 42. The IPA caught by the cup 42 is collected through the cup-side collection pipe 424 for IPA.

When a predetermined time elapses since the start of the high-speed rotation of the spin chuck 41, the rotation of the spin chuck 41 is stopped. At this stage, the IPA is removed from the substrate W, and the substrate W is dried. The dried substrate W is carried out of the processing chamber 44 by the main conveyance robot 3.

Thus, a series of pieces of processing on one substrate W is completed. In the processing unit 4, the series of operations described above is repeated, so that the plurality of substrates W are processed one by one.

### <3. Outline of organic solvent collection part 5 (organic solvent collection apparatus)>

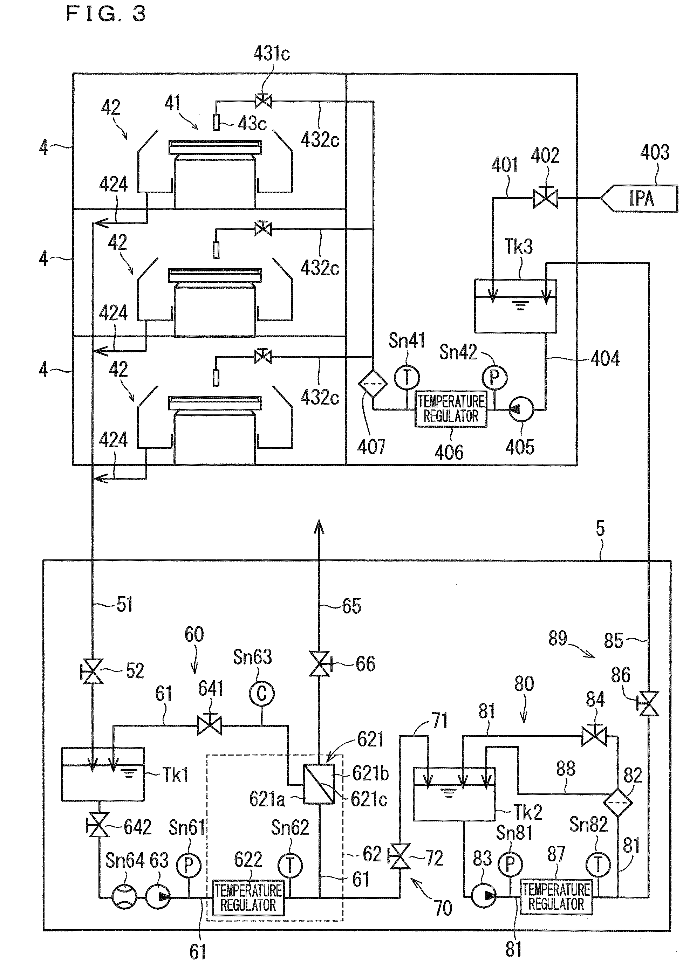

The configuration of the organic solvent collection part 5 will be described with reference to FIG. 3. FIG. 3 is a view schematically illustrating an example of the organic solvent collection part 5 according to the first embodiment. Hereinafter, an outline of the organic solvent collection part 5 will be first described, and then each configuration of the organic solvent collection part 5 will be described in detail.

The organic solvent collection part 5 includes a collection tank Tk1, a dewatering circulator 60, a purification circulator 80, and a liquid sending pipe (second liquid sending pipe) 85. The collection tank Tk1 stores a collection liquid discharged from each processing unit 4. The collection liquid is the mixed liquid of the organic solvent and water. The organic solvent is, for example, an organic solvent having higher volatility than the water or an organic solvent having lower surface tension, and is, as a specific example, isopropyl alcohol (IPA). The mixed liquid from each processing unit 4 flows into the collection tank Tk1 through a collection pipe 51 and is stored in the collection tank Tk1. That is, the mixed liquid is collected in the collection tank Tk1.

The dewatering circulator 60 includes a dewatering circulation pipe 61 and a circulation dewaterer 62. The dewatering circulation pipe 61 is connected to the collection tank Tk1. The dewatering circulation pipe 61 is a pipe through which the mixed liquid is returned from the collection tank Tk1 to the collection tank Tk1.

The circulation dewaterer 62 is interposed in the dewatering circulation pipe 61. For this reason, the mixed liquid flows into the circulation dewaterer 62. The circulation dewaterer 62 separates the water from the flow-in mixed liquid, and causes the water to flow into a separation discharge pipe 65. The separated mixed liquid is continuously circulated through the dewatering circulation pipe 61. By this dewatering, in the dewatering circulation pipe 61, the concentration of the organic solvent in the mixed liquid immediately after the circulation dewaterer 62 (hereinafter, referred to as a solvent concentration) becomes higher than the solvent concentration in the mixed liquid immediately before the circulation dewaterer 62. Because the dewatering circulator 60 circulates the mixed liquid through the dewatering circulation pipe 61, the mixed liquid continues to flow into the circulation dewaterer 62. For this reason, the circulation dewaterer 62 continues to separate the water from the mixed liquid. As a result, the solvent concentration of the mixed liquid during the circulation increases over time. Hereinafter, the mixed liquid in which the solvent concentration is increased to greater than or equal to a predetermined solvent standard value is referred to as a first concentrated liquid. The liquid separated from the mixed liquid by the circulation dewaterer 62 is also referred to as a separation liquid. The separation liquid is almost water. The dewatering circulator 60 generates the first concentrated liquid by the circulation of the mixed liquid.

In the example of FIG. 3, a purification tank Tk2 is provided in the organic solvent collection part 5. The first concentrated liquid from the collection tank Tk1 is supplied to the purification tank Tk2 by a concentrated liquid supply part 70 described later. The purification tank Tk2 stores the first concentrated liquid.

The purification circulator 80 includes a purification circulation pipe 81 and a filter 82. The purification circulation pipe 81 is a circulation pipe that circulates the first concentrated liquid. In the example of FIG. 3, the purification circulation pipe 81 is connected to the purification tank Tk2. The purification circulation pipe 81 is a pipe through which the first concentrated liquid is returned from the purification tank Tk2 to the purification tank Tk2.

The filter 82 is interposed in the purification circulation pipe 81. For this reason, the first concentrated liquid flows into the filter 82. The filter 82 captures impurities in the first concentrated liquid. Due to this capture, an impurity content rate in the first concentrated liquid immediately after the filter 82 is lower than an impurity content rate in the first concentrated liquid immediately before the filter 82. Because the purification circulator 80 circulates the first concentrated liquid through the purification circulation pipe 81, the first concentrated liquid continues to flow into the filter 82. For this reason, the filter 82 continues to capture the impurities from the first concentrated liquid. As a result, the impurity content rate of the first concentrated liquid during the circulation reduces over time. That is, cleanliness of the first concentrated liquid increases over time. Hereinafter, the first concentrated liquid in which the impurity content rate is reduced to less than or equal to a predetermined impurity standard value is also referred to as a recycling liquid. The purification circulator 80 generates the recycling liquid by the circulation of the first concentrated liquid.

A second liquid sending pipe 85 is a pipe through which the recycling liquid generated by the purification circulator 80 flows toward the supply tank Tk3. The recycling liquid in the supply tank Tk3 is supplied to each processing unit 4.

As described above, the organic solvent collection part 5 increases the solvent concentration of the mixed liquid discharged from the processing unit 4 to generate the first concentrated liquid, and then reduces the impurity content rate of the first concentrated liquid to generate the recycling liquid. This recycling liquid is again supplied to the processing unit 4. That is, the substrate processing apparatus 100 recycles the organic solvent in the mixed liquid discharged from the processing unit 4. According to this, an amount of the organic solvent to be discarded can be reduced, and the organic solvent can be more effectively used. That is, the organic solvent collection part 5 contributes to liquid saving.

In addition, in the organic solvent collection part 5, the circulation dewaterer 62 is provided in the dewatering circulation pipe 61, and the filter 82 is provided in the purification circulation pipe 81. That is, the circulation dewaterer 62 and the filter 82 are provided in different circulation pipes. For this reason, the dewatering circulator 60 can circulate the mixed liquid under a first condition corresponding to the circulation dewaterer 62, and the purification circulator 80 can circulate the first concentrated liquid under a second condition corresponding to the filter 82. For example, the first condition and the second condition are various conditions such as a flow rate, a pressure, and a temperature of the fluid flowing through each circulation pipe, and is a temperature condition as a specific example. In this case, as described in detail later, the dewatering circulator 60 can circulate the mixed liquid at a temperature suitable for the circulation dewaterer 62, and the purification circulator 80 can circulate the first concentrated liquid at a temperature suitable for the filter 82. From this viewpoint, an impurity capturing filter may not be provided in the dewatering circulation pipe 61, and a water separation dewaterer may not be provided in the purification circulation pipe 81.

### <3-1. Specific example of organic solvent collection part 5>

### (a1) Collection tank Tk1

The collection tank Tk1 is connected to each cup 42 through the collection pipe 51. That is, the cup 42 (specifically, the cup-side collection pipe 424 connected to the cup 42) is connected to the upstream portion of the collection pipe 51. At this point, for example, the collection pipe 51 is connected to the cup 42 included in each of the plurality of processing units 4 belonging to the same tower. The downstream end of the collection pipe 51 is connected to the collection tank Tk1. A collection valve 52 is interposed in the collection pipe 51. Although not illustrated in FIG. 3, a buffer tank may be provided between the collection tank Tk1 and the processing unit 4.

The collection tank Tk1 can be accommodated in a first accommodation box 50a (see FIG. 1). As an example, the first accommodation box 50a is disposed outside (for example, under a clean room in which the substrate processing apparatus 100 is installed (for example, downstairs)) an outer wall 100a of the substrate processing apparatus 100.

### (b1) Dewatering circulator 60

In the example of FIG. 3, the dewatering circulator 60 includes a pump (dewatering-side liquid sending pump) 63, a first selecting valve 641, and a second selecting valve 642 in addition to the dewatering circulation pipe 61 and the circulation dewaterer 62.

That is, the dewatering circulation pipe 61 forms a circulation path through which the liquid stored in the collection tank Tk1 flows out from the collection tank Tk1 and returns to the collection tank Tk1 again. In the example of FIG. 3, the upstream end of the dewatering circulation pipe 61 is connected to a bottom part of the collection tank Tk1, and the downstream end of the dewatering circulation pipe 61 is connected to an upper part of the collection tank Tk1.

For example, the circulation dewaterer 62 may be a distiller, an atomization separator or a membrane separator. The circulation dewaterer 62 may separate the separation liquid from the mixed liquid with heating of the mixed liquid. In the example of FIG. 3, the circulation dewaterer 62 includes a membrane separator 621 and a dewatering temperature regulator 622. The membrane separator 621 includes a first path 621a, a second path 621b, and a separation membrane 621c. The first path 621a is interposed in the dewatering circulation pipe 61 and constitutes a part of a dewatering circulation path of the dewatering circulator 60. For this reason, the mixed liquid passes through the first path 621a. The separation membrane 621c partitions the first path 621a and the second path 621b. The separation membrane 621c is a membrane that allows the water in the mixed liquid to pass therethrough and substantially blocks the organic solvent. Part of the water in the mixed liquid flowing into the first path 621a passes through the separation membrane 621c and flows into the second path 621b.

The separation membrane 621c may be a zeolite membrane, an organic separation membrane, or a carbon nanotube (CNT) separation membrane. For example, the zeolite membrane has a crystal structure in which (SiOa)⁴⁻ and (AlO₄)⁵⁻ having a tetrahedral structure are mutually connected. For example, the organic separation membrane is an organic film such as polyvinyl alcohol, chitosan, or polyimide. For example, the CNT separation membrane is a membrane that is obtained by adding carbon nanotubes to a membrane of polyamide or the like. Alternatively, a two-dimensional material may be adopted as the material of the separation membrane 621c. The two-dimensional material is a material composed of one layer of atoms, and, for example, may be molybdenum sulfide (MoS₂) or a composite atomic layer compound of a front periodic transition metal (titanium, vanadium, or the like) and a light element (carbon or nitrogen). Alternatively, a metal organic framework (MOF) material or a carbon material (for example, graphene or graphene oxide) may be applied as the material of the separation membrane 621c. At this point, a zeolite membrane is applied as the separation membrane 621c.

The upstream end of the separation discharge pipe 65 is connected to the second path 621b. The separated liquid is discharged to the outside (for example, a waste liquid processing part of factory equipment) through the separation discharge pipe 65. A decompression pump that decompresses the second path 621b may be provided in the separation discharge pipe 65. As illustrated in FIG. 3, a discharge valve 66 is interposed in the separation discharge pipe 65. The separation liquid separated by the circulation dewaterer 62 can slightly contain the organic solvent. A processing part that processes such an organic solvent in the separation liquid may be provided in the separation discharge pipe 65. An example of such processing will be described in the third and fourth embodiments.

The dewatering temperature regulator 622 is provided in the dewatering circulation pipe 61, and adjusts the temperature of the mixed liquid flowing through the dewatering circulation pipe 61. In the example of FIG. 3, the dewatering temperature regulator 622 is provided at a position on the upstream side of the membrane separator 621. The dewatering temperature regulator 622 has a heating function. That is, the dewatering temperature regulator 622 includes a heater. The heater may be an electric resistance heater having an electric heating wire, an optical heater having a light source, or an electronic cooling unit having a Peltier element. When the dewatering temperature regulator 622 heats the mixed liquid, the high-temperature mixed liquid can flow into the membrane separator 621. Because velocity of molecules in the mixed liquid is higher as the temperature is higher, water molecules in the mixed liquid at a high temperature easily pass through the separation membrane 621c. For this reason, the membrane separator 621 can separate the separation liquid from the mixed liquid with higher efficiency. As a result, the dewatering circulator 60 can make the solvent concentration of the mixed liquid greater than or equal to the predetermined solvent reference value in a shorter time.

A dewatering-side liquid sending pump 63 is interposed in the dewatering circulation pipe 61. As an example, the dewatering-side liquid sending pump 63 is provided at the position on the upstream side of the circulation dewaterer 62. The first selecting valve 641 and the second selecting valve 642 are interposed in the dewatering circulation pipe 61. The first selecting valve 641 is provided at the position on the downstream side of the circulation dewaterer 62. The second selecting valve 642 is provided at the position on the upstream side of the dewatering-side liquid sending pump 63.

Various sensors can be interposed in the dewatering circulation pipe 61. For example, a concentration sensor Sn63 that detects the concentration of the organic solvent

(in this case, for example, IPA) in the fluid flowing through the dewatering circulation pipe 61, a flow rate sensor (flow meter) Sn64 that detects the flow rate of the fluid flowing through the dewatering circulation pipe 61, a pressure sensor Sn61 that detects the pressure of the fluid flowing through the dewatering circulation pipe 61, and a temperature sensor Sn62 that detects the temperature of the fluid flowing through the dewatering circulation pipe 61 are interposed in the dewatering circulation pipe 61. For example, the concentration sensor Sn63 is interposed at the position on the downstream side of the circulation dewaterer 62. For example, the flow rate sensor Sn64 is interposed at the position on the upstream side of the dewatering-side liquid sending pump 63. For example, the pressure sensor Sn61 is interposed at the position on the downstream side of the dewatering-side liquid sending pump 63 and the upstream side of the circulation dewaterer 62. For example, the temperature sensor Sn62 is interposed at the position on the downstream side of the dewatering temperature regulator 622 and the upstream side of the membrane separator 621.

### (c1) Purification tank Tk2

The first concentrated liquid is supplied to the purification tank Tk2 by the concentrated liquid supply part 70 described later. For example, the purification tank Tk2 is accommodated in the first accommodation box 50a (see FIG. 1).

### (d1) Concentrated liquid supply part 70

In the example of FIG. 3, the concentrated liquid supply part 70 is provided in the organic solvent collection part 5. The concentrated liquid supply part 70 supplies the first concentrated liquid from the collection tank Tk1 to the purification tank Tk2. The concentrated liquid supply part 70 includes a first liquid sending pipe 71, a first liquid sending valve 72, and the dewatering-side liquid sending pump 63 that is an example of the liquid sending part.

In the example of FIG. 3, the purification tank Tk2 is connected to the dewatering circulation pipe 61 through the first liquid sending pipe 71. That is, the downstream end of the first liquid sending pipe 71 is connected to the purification tank Tk2, and the upstream end of the first liquid sending pipe 71 is connected to the dewatering circulation pipe 61. Specifically, the upstream end of the first liquid sending pipe 71 is connected to the dewatering circulation pipe 61 at the position between the dewatering-side liquid sending pump 63 and the first selecting valve 641. As an example, the upstream end of the first liquid sending pipe 71 is connected to the position on the downstream side of the dewatering temperature regulator 622 (preferably, the downstream side of the temperature sensor Sn2) and the upstream side of the membrane separator 621. Although different from FIG. 3, the upstream end of the first liquid sending pipe 71 may be connected to the position on the downstream side of the circulation dewaterer 62. The upstream end of the first liquid sending pipe 71 is not necessarily connected to the dewatering circulation pipe 61, but may be connected to the collection tank Tk1. In this case, a pump different from the dewatering-side liquid sending pump 63 is provided in the first liquid sending pipe 71. The first liquid sending valve 72 is interposed in the first liquid sending pipe 71.

### (e1) Purification circulator 80

In the example of FIG. 3, the purification circulator 80 includes a pump (purification-side liquid sending pump) 83, a selecting valve 84, and a purification temperature regulator 87 in addition to the purification circulation pipe 81 and the filter 82.

In the example of FIG. 3, the purification circulation pipe 81 forms a circulation path through which the liquid stored in the purification tank Tk2 flows out from the purification tank Tk2 and returns to the purification tank Tk2 again. In the example of FIG. 3, the upstream end of the purification circulation pipe 81 is connected to the bottom part of the purification tank Tk2, and the downstream end of the purification circulation pipe 81 is connected to the upper part of the purification tank Tk2.

The filter 82 captures impurities in the first concentrated liquid flowing through the purification circulation pipe 81. For example, the impurity may be foreign matter generated by processing in the processing unit 4. For example, the filter 82 may be formed in a mesh shape, or may have a configuration in which fine particles are gathered or aggregated. For example, the filter 82 may be made of resin. In the example of FIG. 3, an air venting pipe 88 is connected to the filter 82.

The purification temperature regulator 87 is provided in the purification circulation pipe 81 and adjusts the temperature of the first concentrated liquid flowing through the purification circulation pipe 81. In the example of FIG. 3, the purification temperature regulator 87 is provided at the position on the upstream side of the filter 82. The purification temperature regulator 87 has at least one of a heating function and a cooling function. For example, the purification temperature regulator 87 includes an electronic cooling unit (for example, a Peltier element). The purification temperature regulator 87 heats or cools the first concentrated liquid to adjust the temperature of the first concentrated liquid to the temperature suitable for the filter 82. As described above, when the dewatering temperature regulator 622 heats the mixed liquid, the high-temperature first concentrated liquid is supplied to the purification tank Tk2. In this case, the purification temperature regulator 87 causes the first concentrated liquid having a relatively low temperature to flow into the filter 82 by cooling the first concentrated liquid. That is, the purification temperature regulator 87 adjusts the temperature of the first concentrated liquid to be lower than the temperature of the mixed liquid during the circulation by the dewatering circulator 60. In the above example, because the temperature of the mixed liquid is adjusted to be high by the dewatering temperature regulator 622 in the dewatering circulator 60, the purification temperature regulator 87 adjusts the temperature of the first concentrated liquid to a value closer to the normal temperature than the temperature of the mixed liquid. For example, the normal temperature is a temperature in a room in which the substrate processing apparatus 100 is provided, and is 25°C as a specific example.

When the first concentrated liquid close to the normal temperature flows into the filter 82 in this manner, deformation of the filter 82 due to the heat can be reduced. That is, thermal expansion or thermal contraction of the filter 82 can be reduced. When the filter 82 is greatly deformed by the heat, roughness of the filter 82 may be out of a desired range. When the roughness of the filter 82 is out of the desired range, the capture efficiency of the impurities by the filter 82 is dropped, or the first concentrated liquid hardly passes through the filter 82. In the above example, the purification temperature regulator 87 cools the first concentrated liquid, so that the filter 82 can capture the impurities with high efficiency and can appropriately pass the first concentrated liquid.

A purification-side liquid sending pump 83 is interposed in the purification circulation pipe 81. As an example, the purification-side liquid sending pump 83 is provided at the position on the upstream side of the filter 82. The selecting valve 84 is interposed in the purification circulation pipe 81. As an example, the selecting valve 84 is provided at the position on the downstream side of the filter 82.

Various sensors can be interposed in the purification circulation pipe 81. A pressure sensor Sn81 that detects the pressure of the fluid flowing through the purification circulation pipe 81, a temperature sensor Sn82 that detects the temperature of the fluid flowing through the purification circulation pipe 81, and the like are interposed. For example, the pressure sensor Sn81 is interposed at the position on the downstream side of the purification-side liquid sending pump 83 and the upstream side of the filter 82. For example, the temperature sensor Sn82 is interposed at the position on the downstream side of the purification temperature regulator 87 and the upstream side of the filter 82.

### (f1) Supply tank Tk3

The recycling liquid is supplied to the supply tank Tk3 by a recycling liquid supply part 89 described later. The supply tank Tk3 is accommodated in a second accommodation box 50b (see FIG. 1). As an example, the second accommodation box 50b is disposed inside the outer wall 100a of the substrate processing apparatus 100.

### (g1) recycling liquid supply part 89

In the example of FIG. 3, the recycling liquid supply part 89 is provided in the organic solvent collection part 5. The recycling liquid supply part 89 supplies the recycling liquid to the supply tank Tk3. The recycling liquid supply part 89 includes the second liquid sending pipe 85, a second liquid sending valve 86, and the purification-side liquid sending pump 83 as an example of the liquid sending part.

In the example of FIG. 3, the supply tank Tk3 is connected to the purification circulation pipe 81 through the second liquid sending pipe 85. That is, the downstream end of the second liquid sending pipe 85 is connected to the supply tank Tk3, and the upstream end of the second liquid sending pipe 85 is connected to the purification circulation pipe 81. Specifically, the upstream end of the second liquid sending pipe 85 is connected to the purification circulation pipe 81 at the position between the purification-side liquid sending pump 83 and the selecting valve 84. As an example, the upstream end portion of the second liquid sending pipe 85 is connected to the purification circulation pipe 81 at the position on the upstream side of the filter 82 and the downstream side of the purification temperature regulator 87. The second liquid sending valve 86 is interposed in the second liquid sending pipe 85. The upstream end of the second liquid sending pipe 85 is not necessarily connected to the purification circulation pipe 81, but may be connected to the purification tank Tk2. In this case, a pump different from the purification-side liquid sending pump 83 is provided in the second liquid sending pipe 85.

### (h1) Fresh liquid supply

The supply tank Tk3 is connected to a fresh liquid supply source 403 through a fresh liquid pipe 401. That is, the downstream end of the new liquid pipe 401 is connected to the supply tank Tk3, and the upstream end of the new liquid pipe 401 is connected to the new liquid supply source 403. The new liquid supply source 403 is a supply source of an unused organic solvent (for example, IPA having a concentration greater than or equal to 99.8wt%) that has never been supplied to the substrate W. A new liquid valve 402 is interposed in the new liquid pipe 401.

The supply tank Tk3 is connected to the IPA nozzle 43c through a third liquid sending pipe 404. That is, the supply tank Tk3 is connected to one end side of the third liquid sending pipe 404, and the IPA nozzle 43c (specifically, the IPA pipe 432c connected to the IPA nozzle 43c) is connected to the other end side of the third liquid sending pipe 404. At this point, for example, the third liquid sending pipe 404 is connected to the IPA nozzle 43c included in each of the plurality of processing units 4 belonging to the same tower.

A pump (supply-side liquid sending pump) 405 is interposed in the third liquid sending pipe 404. In the third liquid sending pipe 404, a filter 407 is interposed at a position on the downstream side of the supply-side liquid sending pump 405. In the third liquid sending pipe 404, a temperature regulator 406 is interposed at a position on the upstream side of the filter 407 and on the downstream side of the supply-side liquid sending pump 405.

Various sensors are interposed in the third liquid sending pipe 404. For example, a temperature sensor Sn41 that detects the temperature of the fluid flowing through the third liquid sending pipe 404 is interposed in the third liquid sending pipe 404. For example, the temperature sensor Sn41 is interposed at a position on the upstream side of the filter 407 and on the downstream side of the temperature regulator 406. The temperature regulator 406 adjusts the temperature of the recycling liquid supplied to the processing unit 4 to a predetermined temperature range corresponding to the processing of the substrate W.

### <3-2. Example of operation of organic solvent collection part 5>

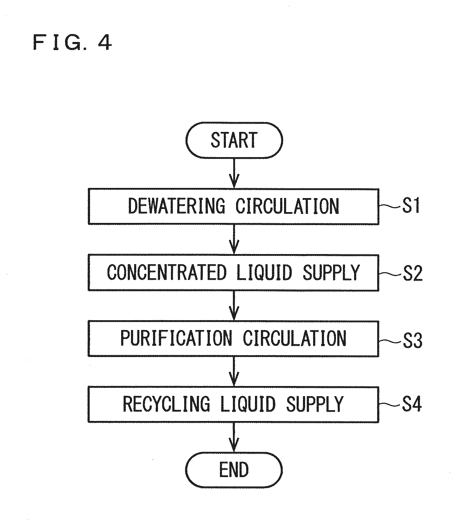

FIG. 4 is a flowchart illustrating an example of the operation of the organic solvent collection part 5. At this point, the mixed liquid is stored in the collection tank Tk1. First, the dewatering circulator 60 circulates the mixed liquid through the dewatering circulation pipe 61 (step S1: dewatering circulation step). Specifically, the controller 6 opens the first selecting valve 641, the second selecting valve 642, and the discharge valve 66 to operate the dewatering-side liquid sending pump 63. Thus, the mixed liquid circulates through the dewatering circulation path including the collection tank Tk1 and the dewatering circulation pipe 61. By this circulation, the mixed liquid continues to pass through the circulation dewaterer 62. For this reason, the circulation dewaterer 62 continues to separate the separation liquid from the mixed liquid, and the separation liquid continues to be discharged to the outside through the separation discharge pipe 65. Consequently, the solvent concentration of the mixed liquid during the circulation increases over time.

At this point, the controller 6 operates the dewatering temperature regulator 622 during the circulation of the mixed liquid. As an example, the controller 6 starts the operation of the dewatering temperature regulator 622 together with the start of circulation of the mixed liquid. For example, the controller 6 may control the dewatering temperature regulator 622 based on the temperature detected by the temperature sensor Sn62. This makes it possible to control the temperature of the mixed liquid with high accuracy. For example, the dewatering temperature regulator 622 heats the mixed liquid to greater than or equal to 40°C. The dewatering temperature regulator 622 may adjust the temperature of the mixed liquid to greater than or equal to 50°C, greater than or equal to 60°C, or greater than or equal to 70°C. Thus, the membrane separator 621 can separate the separation liquid from the mixed liquid with high efficiency.

The controller 6 causes the dewatering circulator 60 to circulate the mixed liquid until the solvent concentration of the mixed liquid during the circulation becomes greater than or equal to the predetermined solvent standard value. For example, the solvent standard value may be greater than or equal to 60 wt%, greater than or equal to 70 wt%, greater than or equal to 80 wt%, greater than or equal to 90 wt%, greater than or equal to 95 wt%, or greater than or equal to 99 wt%. For example, the controller 6 may compare the solvent concentration detected by the concentration sensor Sn63 with the solvent standard value, and may cause the dewatering circulator 60 to stop the circulation when the solvent concentration becomes greater than or equal to the solvent standard value. Specifically, the controller 6 closes the first selecting valve 641, the second selecting valve 642, and the discharge valve 66, and stops the dewatering-side liquid sending pump 63 and the dewatering temperature regulator 622.

By this circulation, the mixed liquid (that is, the first concentrated liquid) having the increased solvent concentration is stored in the collection tank Tk1. The controller 6 may cause the dewatering circulator 60 to stop the circulation with the elapse of a predetermined dewatering time as a trigger. For example, the dewatering time is previously set to greater than or equal to a time required for the solvent concentration to become greater than or equal to the solvent reference value. For example, the dewatering time can be set to greater than or equal to several tens of minutes or greater than or equal to several hours.

In the example of FIG. 4, subsequently, the concentrated liquid supply part 70 supplies the first concentrated liquid from the collection tank Tk1 to the purification tank Tk2 (step S2: concentrated liquid supply step). Specifically, the controller 6 opens the second selecting valve 642 and the first liquid sending valve 72 to operate the dewatering-side liquid sending pump 63. Thus, the first concentrated liquid in the collection tank Tk1 is supplied to the purification tank Tk2 through at least the first liquid sending pipe 71. When a sufficient amount of the first concentrated liquid is supplied to the purification tank Tk2, the controller 6 closes the second selecting valve 642 and the first liquid sending valve 72 to stop the dewatering-side liquid sending pump 63.

Subsequently, the purification circulator 80 circulates the first concentrated liquid through the purification circulation pipe 81 (step S3: purification circulation step). Specifically, the controller 6 opens the selecting valve 84 to operate the purification-side liquid sending pump 83. Thus, the first concentrated liquid circulates through the purification circulation path including the purification tank Tk2 and the purification circulation pipe 81. By this circulation, the first concentrated liquid continues to pass through the filter 82. For this reason, the filter 82 continues to capture the impurities from the first concentrated liquid. Consequently, the impurity content rate of the first concentrated liquid during the circulation reduces over time.

At this point, the controller 6 operates the purification temperature regulator 87 during the circulation of the first concentrated liquid. As an example, the controller 6 starts the operation of the purification temperature regulator 87 together with the start of the circulation of the first concentrated liquid. For example, the controller 6 may control the purification temperature regulator 87 based on the temperature detected by the temperature sensor Sn82. Thus, the temperature of the first concentrated liquid can be controlled with high accuracy. For example, the purification temperature regulator 87 cools the first concentrated liquid to less than or equal to 40°C. The purification temperature regulator 87 may adjust the temperature of the first concentrated liquid to less than or equal to 35°C or less than or equal to 30°C. In addition, the purification temperature regulator 87 may adjust the temperature of the first concentrated liquid to greater than or equal to 20°C. Thus, the filter 82 can capture the impurities in the first concentrated liquid with high efficiency.

For example, the controller 6 circulates the first concentrated liquid in the purification circulator 80 until the impurity content rate of the first concentrated liquid becomes less than or equal to a predetermined impurity reference value. For example, when a predetermined purification time elapses from the start of the circulation of the first concentrated liquid, the controller 6 closes the selecting valve 84 to stop the purification-side liquid sending pump 83 and the purification temperature regulator 87. For example, the purification time is previously set to greater than or equal to a time required for the impurity content rate to become less than or equal to the impurity reference value. A sensor that detects the impurity amount in the first concentrated liquid may be provided, and the controller 6 may cause the purification circulator 80 to stop the circulation when the impurity amount detected by the sensor becomes less than or equal to the reference value. By this circulation, the first concentrated liquid (that is, the recycling liquid) having the reduced impurity content rate is stored in the purification tank Tk2.

Subsequently, the recycling liquid supply part 89 supplies the recycling liquid to the supply tank Tk3 (step S4: recycling liquid supply step). Specifically, the controller 6 opens the second liquid sending valve 86 to operate the purification-side liquid sending pump 83. Thus, the recycling liquid in the purification tank Tk2 is supplied to the supply tank Tk3 through at least the second liquid sending pipe 85.

As described above, the organic solvent collection part 5 separates the separation liquid from the mixed liquid discharged from the processing unit 4 to generate the first concentrated liquid having the increased solvent concentration (step S1), and captures the impurities in the first concentrated liquid to generate the recycling liquid having the reduced impurity content rate of the first concentrated liquid (step S3). Then, the substrate processing apparatus 100 supplies the recycling liquid to the processing unit 4 again (step S4). For this reason, the discard amount of the organic solvent can be reduced, and the organic solvent can be effectively utilized.

In the above example, the circulation dewaterer 62 includes the membrane separator 621. The membrane separator 621 is smaller in size than the distiller, so that the device scale can be reduced.

In addition, in the above-described example, the dewatering temperature regulator 622 heats the mixed liquid, so that the membrane separator 621 can separate the separation liquid from the mixed liquid with high efficiency. On the other hand, the purification temperature regulator 87 cools the first concentrated liquid, so that the filter 82 can capture the impurities in the first concentrated liquid with high efficiency.

In the above example, the purification tank Tk2 is provided, and the first concentrated liquid having the high temperature in the collection tank Tk1 is supplied to the purification tank Tk2 through the first liquid sending pipe 71. Because the first concentrated liquid dissipates the heat during the passage of the first concentrated liquid through the first liquid sending pipe 71, the first concentrated liquid cooled by natural heat dissipation is stored in the purification tank Tk2. For this reason, in the purification circulator 80, the temperature of the first concentrated liquid can be more quickly reduced.

### <Second embodiment>

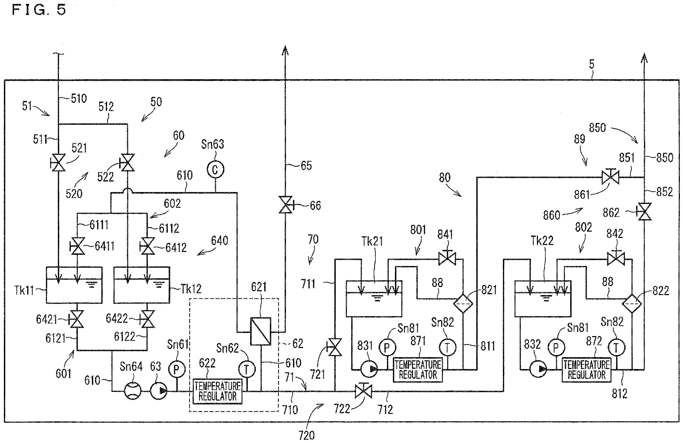

FIG. 5 is a view schematically illustrating an example of an organic solvent collection part 5 according to a second embodiment.

### (a2) Collection tank Tk1

In the second embodiment, a first collection tank Tk11 and a second collection tank Tk 12 are provided as the collection tank Tk1.

### (b2) Collection selector 50

In the second embodiment, a collection selector 50 is provided in the organic solvent collection part 5. The collection selector 50 selects a collection destination of the mixed liquid from the processing unit 4 between the first collection tank Tk11 and the second collection tank Tk12. In the example of FIG. 5, the collection selector 50 includes the collection pipe 51 and a selecting valve part 520. The collection pipe 51 includes a common collection pipe 510, a first branch pipe 511, and a second branch pipe 512. The common collection pipe 510 is connected to each processing unit 4 on the upstream side of the common collection pipe 510. The downstream end of the common collection pipe 510 is connected to the upstream end of the first branch pipe 511 and the upstream end of the second branch pipe 512. The downstream end of the first branch pipe 511 is connected to the first collection tank Tk11, and the downstream end of the second branch pipe 512 is connected to the second collection tank Tk12.

The selecting valve part 520 selects between a state in which the processing unit 4 communicates with the first collection tank Tk11 and a state in which the processing unit 4 communicates with the second collection tank Tk12. In the example of FIG. 3, the selecting valve part 520 includes a first collection valve 521 and a second collection valve 522. The first collection valve 521 is interposed in the first branch pipe 511, and the second collection valve 522 is interposed into the second branch pipe 512.

When the controller 6 opens the first collection valve 521 and closes the second collection valve 522, the mixed liquid is supplied to the first collection tank Tk11. When the controller 6 closes the first collection valve 521 and opens the second collection valve 522, the mixed liquid is supplied to the second collection tank Tk12. As described later, the collection selector 50 alternately selects the collection destination between the first collection tank Tk11 and the second collection tank Tk12.

### (c2) Dewatering circulator 60

The dewatering circulator 60 includes a first dewatering circulation path 601 through the first collection tank Tk11 and a second dewatering circulation path 602 through the second collection tank Tk12. As an example, the first dewatering circulation path 601 is common to the second dewatering circulation path 602 in a common circulation pipe 610 to be described later. The first dewatering circulation path 601 includes the first collection tank Tk11 and a first dewatering circulation pipe 611, and the second dewatering circulation path 602 includes the second collection tank Tk12 and a second dewatering circulation pipe 612. The first dewatering circulation pipe 611 includes a first downstream pipe 6111, the common circulation pipe 610, and a first upstream pipe 6121, and the second dewatering circulation pipe 612 includes a second downstream pipe 6112, the common circulation pipe 610, and a second upstream pipe 6122. For example, the upstream end of the first upstream pipe 6121 is connected to the bottom part of the first collection tank Tk11, and the upstream end of the second upstream pipe 6122 is connected to the bottom part of the second collection tank Tk12. The downstream end of the first upstream pipe 6121 and the downstream end of the second upstream pipe 6122 are connected to the upstream end of the common circulation pipe 610. The downstream end of the common circulation pipe 610 is connected to the upstream end of the first downstream pipe 6111 and the upstream end of the second downstream pipe 6112. For example, the downstream end of the first downstream pipe 6111 is connected to an upper part of the first collection tank Tk11, and the downstream end of the second downstream pipe 6112 is connected to the upper part of the second collection tank Tk12. In this connection relationship, the common circulation pipe 610 is connected between the first downstream pipe 6111 and the first upstream pipe 6121, and is also connected between the second downstream pipe 6112 and the second upstream pipe 6122. For this reason, the common circulation pipe 610 is shared by the first dewatering circulation path 601 and the second dewatering circulation path 602.

In the example of FIG. 5, the circulation dewaterer 62 and the dewatering-side liquid sending pump 63 are interposed in the common circulation pipe 610. A first downstream valve 6411 is interposed in the first downstream pipe 6111, a second downstream valve 6412 is interposed in the second downstream pipe 6112, a first upstream valve 6421 is interposed in the first upstream pipe 6121, and a second upstream valve 6422 is interposed in the second upstream pipe 6122. The first downstream valve 6411, the second downstream valve 6412, the first upstream valve 6421, and the second upstream valve 6422 form a circulation selecting valve part 640 that selects the circulation path of the mixed liquid between the first dewatering circulation path 601 and the second dewatering circulation path 602.

In the example of FIG. 5, various sensors similar to those of the first embodiment are interposed in the common circulation pipe 610.

When the controller 6 opens the first downstream valve 6411 and the first upstream valve 6421 to operate the dewatering-side liquid sending pump 63, the mixed liquid circulates in the first dewatering circulation path 601. By this circulation, the solvent concentration of the mixed liquid can be improved using the circulation dewaterer 62 on the first dewatering circulation path 601. Thus, the mixed liquid (that is, the first concentrated liquid) having the high solvent concentration can be stored in the first collection tank Tk11.

On the other hand, when the controller 6 opens the second downstream valve 6412 and the second upstream valve 6422 to operate the dewatering-side liquid sending pump 63, the mixed liquid circulates in the second dewatering circulation path 602. By this circulation, the solvent concentration of the mixed liquid can be improved using the circulation dewaterer 62 on the second dewatering circulation path 602. Thus, the mixed liquid (that is, the first concentrated liquid) having the high solvent concentration can be stored in the second collection tank Tk12.

As described later, the dewatering circulator 60 circulates the mixed liquid through the second dewatering circulation path 602 during the collection of the mixed liquid by the first collection tank Tk11, and circulates the mixed liquid through the first dewatering circulation path 601 during the collection of the mixed liquid by the second collection tank Tk12.

### (d2) Purification tank Tk2

In the example of FIG. 5, a first purification tank Tk21 and a second purification tank Tk22 are provided as the purification tank Tk2.

### (e2) Concentrated liquid supply part 70

The concentrated liquid supply part 70 supplies the first concentrated liquid from one of the first collection tank Tk 11 and the second collection tank Tk 12 to one of the first purification tank Tk21 and the second purification tank Tk22. As an example, the concentrated liquid supply part 70 has a supply source selecting function of selecting the supply source of the first concentrated liquid between the first collection tank Tk11 and the second collection tank Tk12, and a supply destination selecting function of selecting the supply destination of the first concentrated liquid between the first purification tank Tk21 and the second purification tank Tk22.

The concentrated liquid supply part 70 includes a first liquid sending pipe 71 and a selecting valve part 720 as a supply destination selecting function part. The first liquid sending pipe 71 includes a common liquid sending pipe 710, a first branch pipe 711, and a second branch pipe 712. The upstream end of the common liquid sending pipe 710 is connected to the common circulation pipe 610 as an example, and the downstream end of the common liquid sending pipe 710 is connected to the upstream end of the first branch pipe 711 and the upstream end of the second branch pipe 712. The downstream end of the first branch pipe 711 is connected to the first purification tank Tk21, and the downstream end of the second branch pipe 712 is connected to the second purification tank Tk22.

The selecting valve part 720 selects between the state in which the common circulation pipe 610 communicates with the first purification tank Tk21 and the state in which the common circulation pipe 610 communicates with the second purification tank Tk22. In the example of FIG. 5, the selecting valve part 720 includes a first liquid sending valve 721 and a second liquid sending valve 722. The first liquid sending valve 721 is interposed in the first branch pipe 711, and the second liquid sending valve 722 is interposed in the second branch pipe 712.

In the example of FIG. 5, the supply source selecting function part of the concentrated liquid supply part 70 is formed by the first upstream pipe 6121, the second upstream pipe 6122, a part of the common circulation pipe 610, the first upstream valve 6421, and the second upstream valve 6422. The first upstream valve 6421 and the second upstream valve 6422 form a selecting valve part that selects between the state in which the first collection tank Tk11 communicates with the common circulation pipe 610 and the state in which the second collection tank Tk12 communicates with the common circulation pipe 610.

The controller 6 can change the combination of the supply source and the supply destination by selecting the opening and closing of the first upstream valve 6421, the second upstream valve 6422, the first liquid sending valve 721, and the second liquid sending valve 722. For example, the controller 6 opens the first upstream valve 6421 and the second liquid sending valve 722 to operate the dewatering-side liquid sending pump 63. Thus, the first concentrated liquid is supplied from the first collection tank Tk11 to the second purification tank Tk22.

As described later, the concentrated liquid supply part 70 alternately selects the supply source between the first collection tank Tk11 and the second collection tank Tk12, and alternately selects the supply destination between the first purification tank Tk21 and the second purification tank Tk22.

### (f2) Purification circulator 80

In the example of FIG. 5, the purification circulator 80 includes a first purification circulation path 801 passing through the first purification tank Tk21 and a second purification circulation path 802 passing through the second purification tank Tk22. In the example of FIG. 5, the first purification circulation path 801 includes the first purification tank Tk21 and a first purification circulation pipe 811, and the second purification circulation path 802 includes the second purification tank Tk22 and a second purification circulation pipe 812. For example, the upstream end of the first purification circulation pipe 811 is connected to the bottom part of the first purification tank Tk21, and the downstream end of the first purification circulation pipe 811 is connected to the upper part of the first purification tank Tk21. For example, the upstream end of the second purification circulation pipe 812 is connected to the bottom part of the second purification tank Tk22, and the downstream end of the second purification circulation pipe 812 is connected to the upper part of the second purification tank Tk22.

In the example of FIG. 5, a first filter 821 is interposed in the first purification circulation pipe 811, and a second filter 822 is interposed in the second purification circulation pipe 812. The first filter 821 and the second filter 822 are similar to the filter 82, and capture the impurities in the first concentrated liquid. In the example of FIG. 5, the air venting pipe 88 is connected to each of the first filter 821 and the second filter 822.

In the example of FIG. 5, a first purification temperature regulator 871 is provided in the first purification circulation pipe 811, and a second purification temperature regulator 872 is provided in the second purification circulation pipe 812. The first purification temperature regulator 871 and the second purification temperature regulator 872 are similar to the purification temperature regulator 87, and adjust the temperature of the first concentrated liquid. For example, the first purification temperature regulator 871 and the second purification temperature regulator 872 cool the first concentrated liquid. As an example, the first purification temperature regulator 871 is provided at the position on the upstream side of the first filter 821, and the second purification temperature regulator 872 is provided at the position on the upstream side of the second filter 822.

A first purification-side liquid sending pump 831 is interposed in the first purification circulation pipe 811, and a second purification-side liquid sending pump 832 is interposed in the second purification circulation pipe 812. In the example of FIG. 5, the first purification-side liquid sending pump 831 is provided at the position on the upstream side of the first purification temperature regulator 871, and the second purification-side liquid sending pump 832 is provided at the position on the upstream side of the second purification temperature regulator 872. A first selecting valve 841 is interposed in the first purification circulation pipe 811, and a second selecting valve 842 is interposed in the second purification circulation pipe 812. In the example of FIG. 5, the first selecting valve 841 is provided at the position on the downstream side of the first filter 821, and the second selecting valve 842 is provided at the position on the downstream side of the second filter 822. In the example of FIG. 5, various sensors similar to those of the first embodiment are interposed in each of the first purification circulation pipe 811 and the second purification circulation pipe 812.

When the controller 6 opens the first selecting valve 841 to operate the first purification-side liquid sending pump 831, the first concentrated liquid circulates through the first purification circulation path 801. By this circulation, the impurity content rate of the first concentrated liquid can be reduced using the first filter 821 on the first purification circulation path 801. Thus, the first concentrated liquid (that is, the recycling liquid) having the low impurity content rate can be stored in the first purification tank Tk21.

On the other hand, when the controller 6 opens the second selecting valve 842 to operate the second purification-side liquid sending pump 832, the first concentrated liquid circulates through the second purification circulation path 802. By this circulation, the impurity content rate of the first concentrated liquid can be reduced using the second filter 822 on the second purification circulation path 802. Thus, the first concentrated liquid (that is, the recycling liquid) having the low impurity content rate can be stored in the second purification tank Tk22.

### (g2) recycling liquid supply part 89

The recycling liquid supply part 89 supplies the recycling liquid from each of the first purification tank Tk21 and the second purification tank Tk22 to the supply tank Tk3. The recycling liquid supply part 89 selects the supply source of the recycling liquid between the first purification tank Tk21 and the second purification tank Tk22.

The recycling liquid supply part 89 includes the second liquid sending pipe 85, a selecting valve part 860, and the first purification-side liquid sending pump 831 and the second purification-side liquid sending pump 832 that are examples of the liquid sending part. The second liquid sending pipe 85 includes a common liquid sending pipe 850, a first branch pipe 851, and a second branch pipe 852. As an example, the upstream end of the first branch pipe 851 is connected to the first purification circulation pipe 811 at the position between the first selecting valve 841 and the first purification-side liquid sending pump 831, and the upstream end of the second branch pipe 852 is connected to the second purification circulation pipe 812 at the position between the second selecting valve 842 and the second purification-side liquid sending pump 832. The downstream end of the first branch pipe 851 and the downstream end of the second branch pipe 852 are connected to the upstream end of the common liquid sending pipe 850. For example, the downstream end of the common liquid sending pipe 850 is connected to the upper part of the supply tank Tk3.

The selecting valve part 860 selects between the state in which the first purification tank Tk21 communicates with the supply tank Tk3 and the state in which the second purification tank Tk22 communicates with the supply tank Tk3. In the example of FIG. 5, the selecting valve part 860 includes a first liquid sending valve 861 and a second liquid sending valve 862. The first liquid sending valve 861 is interposed in the first branch pipe 851, and the second liquid sending valve 862 is interposed in the second branch pipe 852.

When the controller 6 opens the first liquid sending valve 861 to operate the first purification-side liquid sending pump 831, the recycling liquid is supplied from the first purification tank Tk21 to the supply tank Tk3. When the controller 6 opens the second liquid sending valve 862 to operate the second purification-side liquid sending pump 832, the recycling liquid is supplied from the second purification tank Tk22 to the supply tank Tk3. As described later, the recycling liquid supply part 89 alternately selects the supply source that supplies the recycling liquid to the supply tank Tk3 between the first purification tank Tk21 and the second purification tank Tk22.

### <Operation of organic solvent collection part 5>

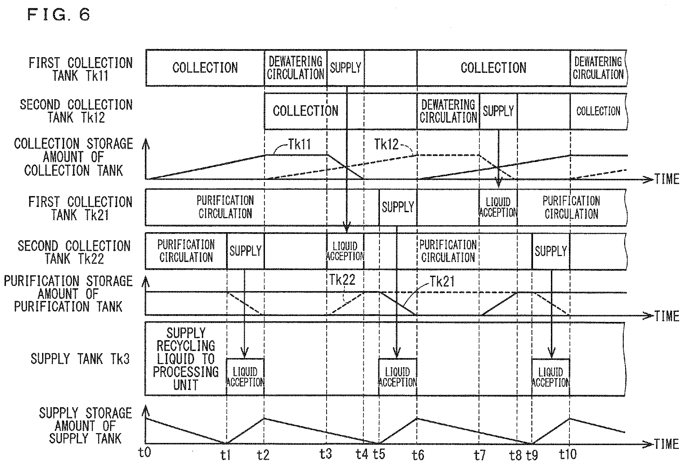

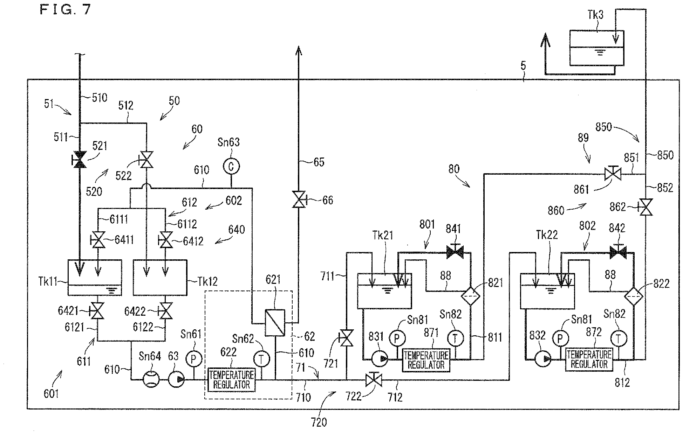

Subsequently, an example of the operation of the organic solvent collection part 5 will be described. FIG. 6 is a view schematically illustrating an example of the operation of the organic solvent collection part 5. FIG. 6 also schematically illustrates an example of a time change in the storage amount (hereinafter, also referred to as a collection storage amount) of the liquid in each of the first collection tank Tkl1 and the second collection tank Tk12. FIG. 6 also schematically illustrates an example of a time change in the storage amount (hereinafter, also referred to as a purification storage amount) of the liquid in each of the first purification tank Tk21 and the second purification tank Tk22. FIG. 6 also schematically illustrates an example of a time change in the storage amount (hereinafter, also referred to as a supply storage amount) of the recycling liquid in the supply tank Tk3. FIGS. 7 to 14 are views illustrating an operation example of the organic solvent collection part 5. In FIGS. 7 to 14, the pipe through which the fluid is flowing is indicated by a thick line, and the open valve is indicated by black.

In the example of FIG. 6, at a time point t0, the first collection tank Tk11 and the second collection tank Tk12 are substantially empty, and the first purification tank Tk21 and the second purification tank Tk22 store the first concentrated liquid (or recycling liquid).

In the example of FIG. 6, first, the first collection tank Tk11 collects the mixed liquid. That is, the collection selector 50 selects the first collection tank Tk11 as the collection destination of the mixed liquid from the processing unit 4. Specifically, as illustrated in FIG. 7, the controller 6 opens the first collection valve 521 and closes the second collection valve 522. For this reason, the collection storage amount of the first collection tank Tk11 increases over time.

In the example of FIG. 6, initially, the circulation is performed through each of the first purification circulation path 801 including the first purification tank Tk21 and the second purification circulation path 802 including the second purification tank Tk22. Specifically, as illustrated in FIG. 7, the controller 6 opens the first selecting valve 841 and the second selecting valve 842 to operate the first purification-side liquid sending pump 831 and the second purification-side liquid sending pump 832. By these circulations, the impurity content rate of the first concentrated liquid reduces with over time in the first purification tank Tk21 and the second purification tank Tk22. Similarly to the first embodiment, the controller 6 may operate the first purification temperature regulator 871 and the second purification temperature regulator 872. Thus, the first filter 821 and the second filter 822 can capture the impurities with high efficiency.

The purification circulator 80 may continue the circulation through the first purification circulation path 801 even after the impurity content rate of the first concentrated liquid in the first purification tank Tk21 becomes lower than or equal to the impurity reference value. That is, the circulation through the first purification circulation path 801 may be continued even after the recycling liquid is stored in the first purification tank Tk21. Thus, the impurity content rate of the recycling liquid in the first purification tank Tk21 can be further reduced. The same applies to the circulation through the second purification circulation path 802.

Because the recycling liquid in the supply tank Tk3 is supplied to the processing unit 4, the supply storage amount of the supply tank Tk3 reduces over time (see the time point t0 to a time point t1). For this reason, the supply storage amount becomes less than or equal to a predetermined supply reference value. The supply reference value may be zero or a value slightly greater than zero. The storage amount stored in each tank can be detected by a storage amount sensor (not illustrated, for example, a liquid level sensor) provided in each tank.

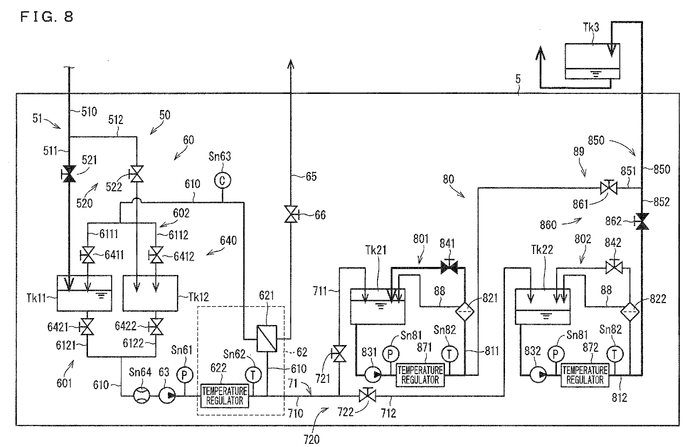

When the supply storage amount becomes less than or equal to the supply reference value, as an example, the purification circulator 80 stops the circulation through the second purification circulation path 802, and the recycling liquid supply part 89 starts to supply the recycling liquid from the second purification tank Tk22 to the supply tank Tk3 (see the time point t1). Specifically, as illustrated in FIG. 8, the controller 6 closes the second selecting valve 842 and opens the second liquid sending valve 862. Thus, the circulation through the second purification circulation path 802 is stopped, and the recycling liquid is supplied from the second purification tank Tk22 to the supply tank Tk3. By this supply, the supply storage amount increases over time, and the purification storage amount of the second purification tank Tk22 reduces over time.

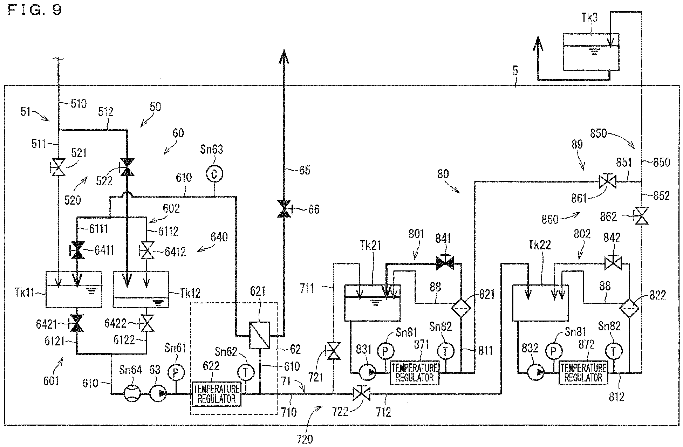

When the sufficient amount of the recycling liquid is supplied to the supply tank Tk3, the recycling liquid supply part 89 stops the supply of the recycling liquid to the supply tank Tk3 (see a time point t2). Specifically, as illustrated in FIG. 9, the controller 6 closes the second liquid sending valve 862 and stops the second purification-side liquid sending pump 832. As an example, the second purification tank Tk22 is substantially empty at the time point t2. By this supply stop, the supply storage amount reduces again over time.

On the other hand, because the mixed liquid from the processing unit 4 is supplied to the first collection tank Tk11, the collection storage amount of the first collection tank Tk11 increases over time. When the collection storage amount of the first collection tank Tk11 becomes greater than or equal to the predetermined circulation reference value, the collection selector 50 selects the collection destination of the mixed liquid from the processing unit 4 from the first collection tank Tk11 to the second collection tank Tk12. In FIG. 6, the collection selector 50 selects the collection destination to the second collection tank Tk12 at the time point t2. Specifically, as illustrated in FIG. 9, the controller 6 closes the first collection valve 521 and opens the second collection valve 522. Thus, the collection storage amount of the second collection tank Tk12 increases over time.

When the collection selector 50 selects the collection destination of the mixed liquid to the second collection tank Tk12, the dewatering circulator 60 starts to circulate the mixed liquid through the first dewatering circulation path 601 including the first collection tank Tk11 (see the time point t2). That is, the dewatering circulator 60 circulates the mixed liquid through the dewatering circulation path including the collection tank, which is not used for collection, in the first collection tank Tk11 and the second collection tank Tk12. Specifically, as illustrated in FIG. 9, the controller 6 opens the first downstream valve 6411, the first upstream valve 6421, and the discharge valve 66 to operate the dewatering-side liquid sending pump 63. By this circulation, the solvent concentration of the mixed liquid in the first collection tank Tk11 increases over time. Similarly to the first embodiment, the controller 6 may operate the dewatering temperature regulator 622 during the circulation. Thus, the circulation dewaterer 62 can separate the separation liquid from the mixed liquid with high efficiency.

As described above, the mixed liquid is circulated through the first dewatering circulation path 601 during the collection of the mixed liquid by the second collection tank Tk12. Conversely, during this circulation, the mixed liquid from the processing unit 4 is not supplied to the first collection tank Tk11. For this reason, the mixed liquid having the low solvent concentration can be avoided from flowing into the mixed liquid during the circulation. In addition, because the temperature of the mixed liquid from the processing unit 4 is not necessarily within the range suitable for the separation by the circulation dewaterer 62, when the mixed liquid from the processing unit 4 flows into the mixed liquid during the circulation, the temperature of the mixed liquid during the circulation may fluctuate and become out of the range. In this case, there is a possibility that the separation efficiency is dropped. According to the above example, such a drop in the separation efficiency can also be avoided.

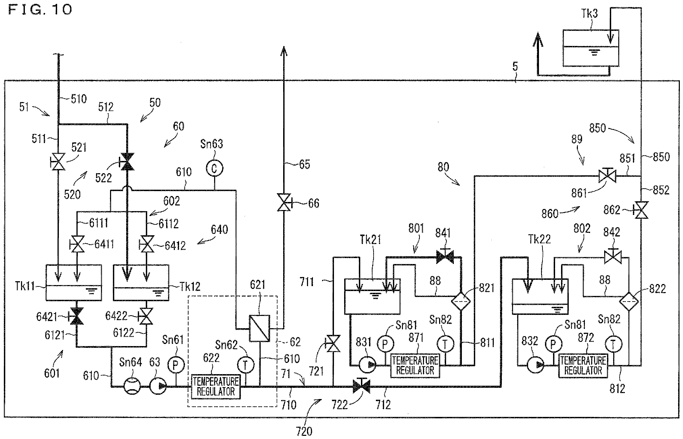

When the solvent concentration of the mixed liquid in the first collection tank Tk11 becomes greater than or equal to the solvent reference value, the dewatering circulator 60 stops the circulation (see a time point t3). Subsequently, the concentrated liquid supply part 70 supplies the first concentrated liquid from the first collection tank Tk11 to the second purification tank Tk22 having the storage amount less than or equal to a predetermined replenishment reference value. For example, the replenishment reference value may be previously set to zero or a value slightly greater than zero. Specifically, as illustrated in FIG. 10, the controller 6 closes the first downstream valve 6411 and opens the second liquid sending valve 722. The circulation of the mixed liquid is stopped by closing the first downstream valve 6411. At this point, because the dewatering-side liquid sending pump 63 is continuously operated, the first concentrated liquid is supplied from the first collection tank Tk11 to the second purification tank Tk22. By this supply, the collection storage amount of the first collection tank Tk11 reduces over time, and the purification storage amount of the second purification tank Tk22 increases over time.

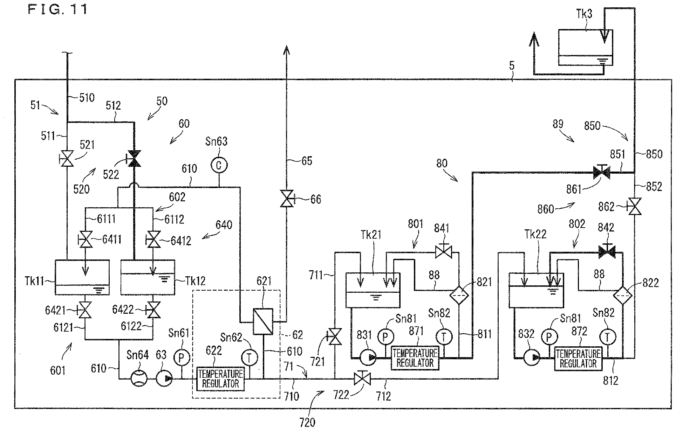

When the sufficient amount of the first concentrated liquid is supplied to the second purification tank Tk22, the concentrated liquid supply part 70 stops the supply of the first concentrated liquid to the second purification tank Tk22 (see a time point t4). Specifically, as illustrated in FIG. 11, the controller 6 closes the second liquid sending valve 722 to stop the dewatering-side liquid sending pump 63. In the example of FIG. 6, the first collection tank Tk11 is substantially empty at a time point t4.

Subsequently, the purification circulator 80 starts the circulation of the first concentrated liquid through the second purification circulation path 802 including the second purification tank Tk22 (see the time point t4). Specifically, as illustrated in FIG. 11, the controller 6 opens the second selecting valve 842 to operate the second purification-side liquid sending pump 832. By this circulation, the impurity content rate of the first concentrated liquid in the second purification tank Tk22 reduces over time. Similarly to the first embodiment, the controller 6 may operate the second purification temperature regulator 872. The purification circulator 80 may start the circulation during the supply of the first concentrated liquid to the second purification tank Tk22 (that is, from the time point t3 to the time point t4).

When the supply storage amount becomes less than or equal to the supply reference value again, the recycling liquid supply part 89 starts to supply the recycling liquid to the supply tank Tk3 (see a time point t5). At this point, it is assumed that the impurity content rate of the first concentrated liquid in the second purification tank Tk22 does not yet become less than or equal to the impurity reference value. For this reason, the recycling liquid supply part 89 supplies the recycling liquid from the first purification tank Tk21 to the supply tank Tk3. Prior to this supply, the purification circulator 80 can stop the circulation through the first purification circulation path 801. Specifically, as illustrated in FIG. 11, the controller 6 closes the first selecting valve 841 and opens the first liquid sending valve 861. By this supply, the purification storage amount of the first purification tank Tk21 reduces over time, and the supply storage amount increases over time.

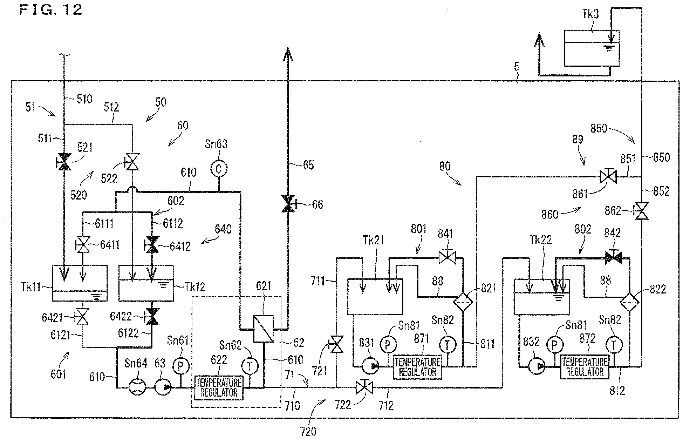

When the sufficient amount of the recycling liquid is supplied to the supply tank Tk3, the recycling liquid supply part 89 stops the supply of the recycling liquid to the supply tank Tk3 (see a time point t6). Specifically, as illustrated in FIG. 12, the controller 6 closes the first liquid sending valve 861 and stops the first purification-side liquid sending pump 831. In the example of FIG. 6, at the time point t6, the first purification tank Tk21 is substantially empty. By this supply stop, the supply storage amount reduces again over time.

On the other hand, when the collection storage amount of the second collection tank Tk12 becomes greater than or equal to the predetermined circulation reference value, the collection selector 50 selects the collection destination of the mixed liquid from the processing unit 4 from the second collection tank Tk12 to the first collection tank Tk11. In FIG. 6, the collection selector 50 selects the collection destination to the first collection tank Tk11 at the time point t6. Specifically, as illustrated in FIG. 12, the controller 6 opens the first collection valve 521 and closes the second collection valve 522. Thus, the collection storage amount of the first collection tank Tk11 increases over time.

As described above, the collection selector 50 selects the collection destination to the first collection tank Tk11 at the time point t6. At this point, after the time point t4 at which the first concentrated liquid in the first collection tank Tk11 is completely supplied to the second purification tank Tk22, the first collection tank Tk11 accepts the mixed liquid from the processing unit 4. In short, the organic solvent collection part 5 ends the generation of the first concentrated liquid in the first collection tank Tk11 (from the time point t2 to the time point t3) and the supply of the first concentrated liquid from the first collection tank Tk11 to the second purification tank Tk22 (from the time point t3 to the time point t4) by the time point t6. In other words, the capacity of the circulation dewaterer 62, the pressure (target value), the flow rate (target value), and the temperature (target value) of the mixed liquid during the circulation, and the flow rate (target value) of the mixed liquid supplied to the second purification tank Tk22 can be set so as to satisfy this condition.

Subsequently, the dewatering circulator 60 starts the circulation of the mixed liquid through the second dewatering circulation path 602 including the second collection tank Tk12 (see the time point t6). Specifically, as illustrated in FIG. 12, the controller 6 opens the second downstream valve 6412, the second upstream valve 6422, and the discharge valve 66 to operate the dewatering-side liquid sending pump 63. By this circulation, the solvent concentration of the mixed liquid in the second collection tank Tk12 increases over time. Similarly to the first embodiment, the controller 6 may operate the dewatering temperature regulator 622.

As described above, the mixed liquid circulates through the second dewatering circulation path 602 during the collection of the mixed liquid by the first collection tank Tkl 1. In other words, during this circulation, the mixed liquid from the processing unit 4 is not supplied to the second collection tank Tk12. For this reason, the mixed liquid from the processing unit 4 can be avoided flowing into the mixed liquid during the circulation. Consequently, the drop in the solvent concentration of the mixed liquid during the circulation and a temperature fluctuation caused by the inflow can be avoided.

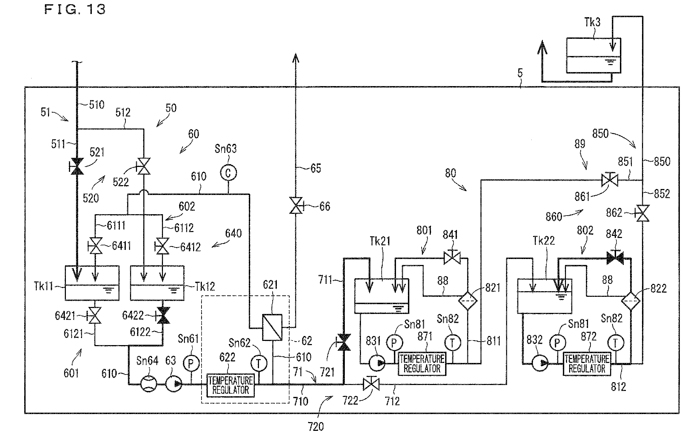

When the solvent concentration of the mixed liquid in the second collection tank Tk12 becomes greater than or equal to the solvent reference value, the dewatering circulator 60 stops the circulation through the second dewatering circulation path 602 (see a time point t7). Subsequently, the concentrated liquid supply part 70 starts to supply the first concentrated liquid from the second collection tank Tk12 to the first purification tank Tk21 in which the storage amount is less than or equal to the replenishment reference value. Specifically, as illustrated in FIG. 13, the controller 6 closes the second downstream valve 6412 and opens the first liquid sending valve 721. The circulation of the mixed liquid is stopped by closing the second downstream valve 6412. At this point, because the dewatering-side liquid sending pump 63 is continuously operated, the first concentrated liquid is supplied from the second collection tank Tk12 to the first purification tank Tk21. By this supply, the collection storage amount of the second collection tank Tk12 reduces over time, and the purification storage amount of the first purification tank Tk21 increases over time.

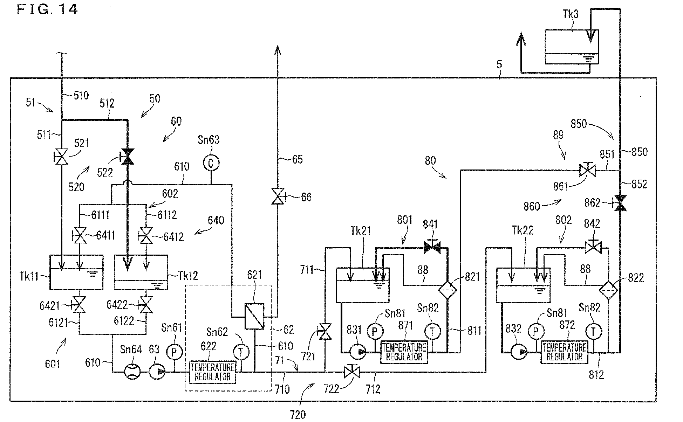

When the sufficient amount of the first concentrated liquid is supplied to the first purification tank Tk21, the concentrated liquid supply part 70 stops the supply of the first concentrated liquid to the first purification tank Tk21 (see a time point t8). Specifically, as illustrated in FIG. 14, the controller 6 closes the first liquid sending valve 721 and stops the dewatering-side liquid sending pump 63. In the example of FIG. 6, the second collection tank Tk12 is substantially empty at the time point t8.

Subsequently, the purification circulator 80 starts the circulation of the first concentrated liquid through the first purification circulation path 801 including the first purification tank Tk21 (see the time point t8). Specifically, as illustrated in FIG. 14, the controller 6 opens the first selecting valve 841 to operate the first purification-side liquid sending pump 831. By this circulation, the impurity content rate of the first concentrated liquid in the first purification tank Tk21 reduces over time. Similarly to the first embodiment, the controller 6 may operate the first purification temperature regulator 871. The purification circulator 80 may start the circulation during the supply of the first concentrated liquid to the first purification tank Tk21 (that is, from the time point t7 to the time point t8).

When the supply storage amount becomes less than or equal to the supply reference value again, the recycling liquid supply part 89 starts to supply the recycling liquid to the supply tank Tk3 (see a time point t9). At this point, it is assumed that the impurity content rate of the first concentrated liquid in the first purification tank Tk21 does not yet become less than or equal to the impurity reference value. On the other hand, the second purification tank Tk22 stores the recycling liquid having the impurity content rate lower than or equal to the impurity reference value. That is, at this point, the recycling liquid is stored in the second purification tank Tk22 by the time point t9. In short, from the start of the circulation through the second purification circulation path 802 (time point t4) to the time point t9 at which the storage amount in the supply tank Tk3 becomes less than or equal to the supply reference value for the second time, the impurity content rate of the first concentrated liquid in the second purification tank Tk22 becomes less than or equal to the impurity reference value. Conversely, the capacity of the second filter 822, and the pressure (target value), the flow rate (target value), and the temperature (target value) of the first concentrated liquid during circulation may previously be set such that this condition is satisfied.

The recycling liquid supply part 89 supplies the recycling liquid from the second purification tank Tk22 to the supply tank Tk3 while the purification circulator 80 stops the circulation through the second purification circulation path 802. Specifically, as illustrated in FIG. 14, the controller 6 closes the second selecting valve 842 and opens the second liquid sending valve 862. By this supply, the purification storage amount of the second purification tank Tk22 reduces over time, and the supply storage amount increases over time.

When the sufficient amount of the recycling liquid is supplied to the supply tank Tk3, the recycling liquid supply part 89 stops the supply of the recycling liquid to the supply tank Tk3 (see a time point t10).

On the other hand, when the collection storage amount of the first collection tank Tk11 becomes greater than or equal to the predetermined circulation reference value, the collection selector 50 selects the collection destination of the mixed liquid from the processing unit 4 from the first collection tank Tk11 to the second collection tank Tk12 again. In the example of FIG. 6, the collection selector 50 selects the collection destination to the second collection tank Tk12 at the time point t10. Specifically, as illustrated in FIG. 14, the controller 6 closes the first collection valve 521 and opens the second collection valve 522. Thus, the collection storage amount of the second collection tank Tk12 increases over time.

As described above, the collection selector 50 selects the collection destination to the second collection tank Tk12 at the time point t10. At this point, after the time point t8 at which the first concentrated liquid in the second collection tank Tk12 is completely supplied to the first purification tank Tk21, the second collection tank Tk12 accepts the mixed liquid from the processing unit 4. In short, the organic solvent collection part 5 ends the generation of the first concentrated liquid in the second collection tank Tk12 (from the time point t6 to the time point t7) and the supply of the first concentrated liquid from the second collection tank Tk12 to the first purification tank Tk21 (from the time point t7 to the time point t8) by the time point t10. In other words, the capacity of the circulation dewaterer 62, the pressure (target value), the flow rate (target value), and the temperature (target value) of the mixed liquid during the circulation, and the flow rate (target value) of the mixed liquid supplied to the first purification tank Tk21 can be set so as to satisfy this condition.

Thereafter, the organic solvent collection part 5 repeats the above-described operation.

As described above, in the second embodiment, the first collection tank Tk11 and the second collection tank Tk12 are provided, and the collection selector 50 alternately selects the collection destination of the mixed liquid from the processing unit 4 between the first collection tank Tk11 and the second collection tank Tk12. Moreover, the organic solvent collection part 5 generates the first concentrated liquid in the second collection tank Tk11 and supplies the first concentrated liquid from the second collection tank Tk12 to the first purification tank Tk21 during the collection of the mixed liquid in the first collection tank Tk12. Similarly, the organic solvent collection part 5 generates the first concentrated liquid in the first collection tank Tk12 and supplies the first concentrated liquid from the first collection tank Tk11 to the second purification tank Tk22 during the collection of the mixed liquid in the second collection tank Tk11.

Thus, the organic solvent collection part 5 can generate the first concentrated liquid while constantly continuing the collection of the mixed liquid from the processing unit 4. Moreover, the mixed liquid from the processing unit 4 does not flow into the mixed liquid during the circulation. For this reason, the drop in the solvent concentration of the mixed liquid during the circulation caused by the inflow can be avoided. In addition, the temperature fluctuation of the mixed liquid during the circulation caused by the inflow can also be avoided. Consequently, the circulation dewaterer 62 can separate the separation liquid from the mixed liquid with high efficiency.

In the above example, in the dewatering circulator 60, the circulation dewaterer 62 is provided in the common circulation pipe 610. For this reason, the circulation dewaterer 62 can be used in both the first dewatering circulation path 601 and the second dewatering circulation path 602. Consequently, the apparatus scale and the manufacturing cost can be reduced.

This is particularly advantageous for the substrate processing apparatus 100 in which the mixed liquid is continuously discharged from the plurality of processing units 4. That is, because any one of the first collection tank Tk11 and the second collection tank Tk12 is always used for collecting the mixed liquid from the processing unit 4, it is not necessary to perform the circulation through the first dewatering circulation path 601 and the circulation through the second dewatering circulation path 602 in parallel, but these circulations may be alternately performed. For this reason, even when the circulation dewaterer 62 is used in both the first dewatering circulation path 601 and the second dewatering circulation path 602, the circulation of the first dewatering circulation path 601 and the circulation of the second dewatering circulation path 602 can be appropriately implemented.

In the above example, the first purification tank Tk21 and the second purification tank Tk22 are provided, and the purification circulator 80 includes the first filter 821 provided in the first purification circulation path 801 and the second filter 822 provided in the second purification circulation path 802. Thus, the purification circulator 80 can perform the circulation of the first concentrated liquid through the first purification circulation path 801 and the circulation of the first concentrated liquid through the second purification circulation path 802 in parallel (for example, from the time point t4 to the time point t5 and from the time point t8 to the time point t9). For this reason, the circulation time of the first concentrated liquid can be lengthened, and the recycling liquid having the lower impurity content rate can be generated.

### <Third embodiment>

The circulation dewaterer 62 may slightly separate the organic solvent in the mixed liquid when separating the water from the mixed liquid. For example, although the separation membrane 621c almost blocks the organic solvent, a small part of the organic solvent in the mixed liquid can actually pass through the separation membrane 621c. In other words, it can be said that the separation membrane 621c is a membrane that allows the water to pass more easily than the organic solvent. That is, the ratio of the amount of water passing through the separation membrane 621c to the amount of water flowing into the membrane separator 621 is greater than the ratio of the amount of the organic solvent passing through the separation membrane 621c to the amount of the organic solvent flowing into the membrane separator 621.

As described above, the separation liquid can contain a slight amount of the organic solvent. Accordingly, in the third embodiment, the organic solvent contained in the separation liquid is intended to be processed.

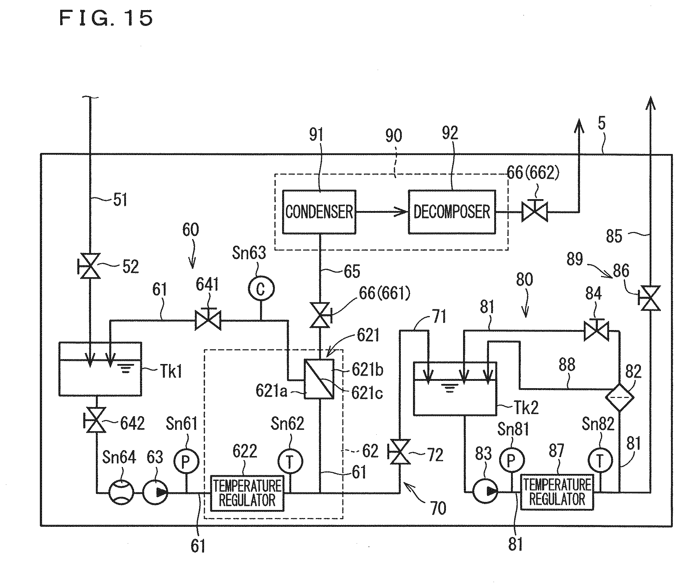

FIG. 15 is a view schematically illustrating an example of an organic solvent collection part 5 according to the third embodiment. The organic solvent collection part 5 according to the third embodiment is different from that of the first or second embodiment in the existence of a separation liquid processing part 90.

The separation liquid processing part 90 is interposed in the separation discharge pipe 65. The separation liquid flows into the separation liquid processing part 90. The separation liquid slightly contains the organic solvent. The solvent concentration of the separation liquid is smaller than the solvent concentration of the mixed liquid. The separation liquid processing part 90 processes the separation liquid to further reduce the solvent concentration of the separation liquid. In the example of FIG. 15, the separation liquid processing part 90 includes a decomposer 92. The decomposer 92 is interposed in the separation discharge pipe 65. The separation liquid flows into the decomposer 92. The decomposer 92 decomposes the organic solvent in the separation liquid. Specifically, the decomposer 92 decomposes the organic solvent into the water and other components. For example, the other components include carbon dioxide.

For example, the decomposer 92 may decompose the organic solvent by electrolysis. For example, the decomposer 92 includes a tank that stores the inflow separation liquid, a pair of electrodes immersed in the separation liquid in the tank, and a power source that applies voltage between the pair of electrodes. The power source applies the voltage between the pair of electrodes to decompose the organic solvent in the separation liquid.

A liquid vapor can also flow through each pipe. For example, a water vapor and a vapor of the organic solvent can flow through each pipe. When the organic solvent is more volatile than the water, the vapor of the organic solvent is likely to be generated in the pipe. In particular, in the example of FIG. 15, the circulation dewaterer 62 includes the dewatering temperature regulator 622, and separates the separation liquid from the mixed liquid with heating of the mixed liquid. For this reason, the temperatures of the mixed liquid and the separation liquid are relatively high. Because the mixed liquid and the separation liquid evaporate as the temperature increases, for example, the vapor of the mixed liquid flows in the dewatering circulation pipe 61, and as a result, the vapor of the separation liquid flows into the upstream end of the separation discharge pipe 65. The higher the temperature, the larger the proportion of the vapor in the separation liquid. Hereinafter, the separation liquid and the vapor of the separation liquid are collectively referred to as a separation fluid. A main component of the separation fluid is the water.

Accordingly, in the example of FIG. 15, the separation liquid processing part 90 also includes a condenser 91. The condenser 91 is interposed in the separation discharge pipe 65 at the position on the upstream side of the decomposer 92. The separation fluid flows into the condenser 91. The condenser 91 condenses at least the vapor of the organic solvent. That is, the vapor of the organic solvent changes into liquid. For example, the condenser 91 includes a heat exchanger and a cooling source. The heat exchanger is interposed in the separation discharge pipe 65. The cooling source cools the heat exchanger. For example, the cooling source may supply a refrigerant to the heat exchanger. The refrigerant may be gas or liquid. Alternatively, the cooling source may be the electronic cooling unit provided in the heat exchanger. The separation fluid passing through the heat exchanger is deprived of heat by the heat exchanger and is cooled. Thus, at least the vapor of the organic solvent is changed into the liquid. The condenser 91 may condense most or all of the vapor of the organic solvent. The condenser 91 may also condense most or all of the water vapor.

This makes it possible to increase the amount of the organic solvent in the liquid flowing into the decomposer 92 while reducing the amount of the vapor of the organic solvent flowing into the decomposer 92. For this reason, the decomposer 92 can more reliably electrolyze the organic solvent. The decomposer 92 may decompose most or all of the organic solvent (liquid).

In the example of FIG. 15, a first discharge valve 661 and a second discharge valve 662 are provided as the discharge valve 66. The first discharge valve 661 is interposed in the separation discharge pipe 65 at the position on the upstream side of the separation liquid processing part 90, and the second discharge valve 662 is interposed in the separation discharge pipe 65 at the position on the downstream side of the separation liquid processing part 90.

The controller 6 operates the separation liquid processing part 90 during the circulation of the mixed liquid by the dewatering circulator 60. Specifically, the controller 6 operates the cooling source of the condenser 91 and operates the power source of the decomposer 92. For example, the controller 6 starts the operation of the separation liquid processing part 90 together with the start of the circulation of the mixed liquid by the dewatering circulator 60, and stops the operation of the separation liquid processing part 90 together with the stop of the circulation of the mixed liquid by the dewatering circulator 60.

When the third embodiment is applied to the second embodiment, the controller 6 operates the separation liquid processing part 90 during each of the circulation of the mixed liquid through the first dewatering circulation path 601 and the circulation of the mixed liquid through the second dewatering circulation path 602.

When the decomposer 92 has a tank, the decomposer 92 may electrolyze the separation liquid when the sufficient amount of the separation liquid is stored in the tank. In this case, the decomposition of the organic solvent is not necessarily performed during the circulation of the mixed liquid.

As described above, in the third embodiment, the separation liquid processing part 90 decomposes the organic solvent in the separation liquid. For this reason, the discharge processing part (for example, the discharge processing part of factory equipment) of the discharge destination easily processes the separation liquid. In the above example, the condenser 91 condenses the vapor of the organic solvent, and then the decomposer 92 electrolyzes the organic solvent in the separation liquid. For this reason, the amount of the organic solvent flowing out to the downstream side from the separation liquid processing part 90 can be more reliably reduced.

### <Fourth embodiment>

### <First example>

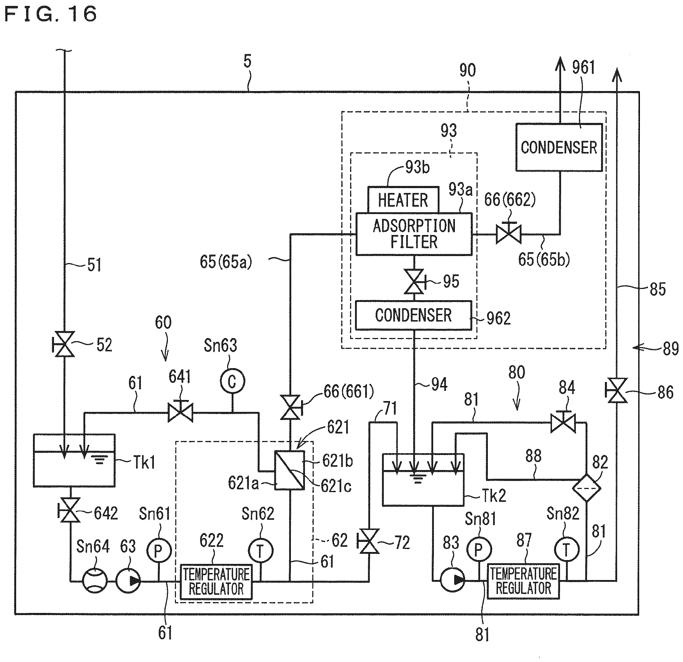

FIG. 16 is a view schematically illustrating a first example of an organic solvent collection part 5 according to a fourth embodiment. The organic solvent collection part 5 according to a first example of the fourth embodiment is different from the organic solvent collection part 5 according to the third embodiment in an internal configuration of the separation liquid processing part 90.

The separation liquid processing part 90 according to the fourth embodiment separates the organic solvent from the separation fluid. Then, the separation liquid processing part 90 discharges the separation fluid after the organic solvent is separated to the outside, and joins the separated organic solvent in the first concentrated liquid. In the example of FIG. 16, the separation liquid processing part 90 supplies the separated organic solvent to the purification tank Tk2.

The separation liquid processing part 90 can actually separate the water from the separation fluid. Accordingly, hereinafter, the liquid separated from the separation fluid by the separation liquid processing part 90 is also referred to as a second concentrated liquid. The second concentrated liquid may contain not only the organic solvent but also slightly the water. The main component of the second concentrated liquid is the organic solvent, and the solvent concentration is high. The second concentrated liquid may be composed only of the organic solvent.

In the example of FIG. 16, the separation liquid processing part 90 includes a separation dewaterer 93 and a return pipe 94. The separation dewaterer 93 is interposed in the separation discharge pipe 65. Hereinafter, a portion of the separation discharge pipe 65 on the upstream side of the separation dewaterer 93 is referred to as an upstream discharge pipe 65a, and a portion on the downstream side is referred to as a downstream discharge pipe 65b. The separation dewaterer 93 is also connected to the upstream end of the return pipe 94. The separation fluid flows into the separation dewaterer 93 through the upstream discharge pipe 65a. This separation fluid can contain the slight amount of the organic solvent. The separation dewaterer 93 separates the second concentrated liquid from the separation fluid. The separation dewaterer 93 causes the separation fluid (mainly the water) after the second concentrated liquid is separated to flow to the downstream discharge pipe 65b, and causes the second concentrated liquid to flow to the return pipe 94. The return pipe 94 is a pipe through which the second concentrated liquid is joined in the first concentrated liquid. In the example of FIG. 16, the downstream end of the return pipe 94 is connected to the purification tank Tk2.

At this point, an example of a difference between the circulation dewaterer 62 and the separation dewaterer 93 will be described. The circulation dewaterer 62 and the separation dewaterer 93 are different from each other in the lower limit value of the application range of the solvent concentration. The application range of the solvent concentration is the application range of the solvent concentration of the fluid flowing into the dewaterer. When the fluid having the solvent concentration below the lower limit of this application range flows into the dewaterer, a failure of the dewaterer may be generated. For example, the dewaterer cannot sufficiently separate the water from the fluid. The lower limit value of the circulation dewaterer 62 is higher than the lower limit value of the separation dewaterer 93. That is, the circulation dewaterer 62 is a dewaterer for a higher solvent concentration compared with the separation dewaterer 93. In other words, the separation dewaterer 93 is a dewaterer for a lower solvent concentration compared with the circulation dewaterer 62. When the separation membrane 621c is a zeolite membrane, the lower limit value of the zeolite membrane may be about 50 wt%. For example, when the mixed liquid having the low solvent concentration flows into the zeolite membrane, there is a risk that the zeolite membrane is damaged.

For example, the separation dewaterer 93 includes an adsorption filter 93a that captures the organic solvent, a heater 93b that heats the adsorption filter 93a, and a condenser 961. For example, the adsorption filter 93a is activated carbon. The adsorption filter 93a captures the organic solvent by an adsorption action. The adsorption filter 93a separates the organic solvent from the separation fluid by capturing the organic solvent in the separation fluid. The lower limit value of the application range of the solvent concentration for the adsorption filter 93a is lower than the lower limit value of the separation membrane 621c, and, for example, may be less than or equal to 10 wt%, less than or equal to 5 wt%, less than or equal to 2 wt%, less than or equal to 1 wt%, or less than or equal to 0.1 wt%.

The heater 93b heats the adsorption filter 93a in the state in which the separation fluid does not flow through the adsorption filter 93a, and causes the adsorption filter 93a to release the organic solvent (for example, the vapor). The organic solvent released from the adsorption filter 93a flows into the return pipe 94. The heater 93b may be an electric resistance heater having an electric heating wire or an optical heater having a light source. The heater 93b may include a heating pipe having the downstream end connected to the adsorption filter 93a, a gas supply source that supplies the gas through the heating pipe, and a heater that heats the gas flowing through the heating pipe. When the high-temperature gas (for example, nitrogen) is supplied to the adsorption filter 93a, the adsorption filter 93a is heated by the heat exchange between the gas and the adsorption filter 93a. The adsorption filter 93a releases the organic solvent, so that the adsorption capacity of the adsorption filter 93a can be restored.

The second discharge valve 662 is interposed in the downstream discharge pipe 65b, and a return valve 95 is interposed in the return pipe 94.

In the example of FIG. 16, the separation liquid processing part 90 also includes the condenser 961. The condenser 961 is interposed in the downstream discharge pipe 65b. As an example, the condenser 961 is provided at the position on the downstream side of the second discharge valve 662. The condenser 961 condenses the vapor (mainly the water vapor) contained in the separation fluid (mainly the water and the water vapor) that passes through the separation dewaterer 93. For example, the configuration of the condenser 961 is similar to that of the condenser 91.

The condenser 962 is interposed in the return pipe 94. As an example, the condenser 962 is provided at the position on the downstream side of the return valve 95. The condenser 962 condenses the vapor of the organic solvent from the separation dewaterer 93. For example, the configuration of the condenser 962 is similar to that of the condenser 91.

The controller 6 circulates the mixed liquid in the dewatering circulator 60 in the state in which the first discharge valve 661 and the second discharge valve 662 are opened and the return valve 95 is closed. During the circulation of the mixed liquid, the separation fluid from the circulation dewaterer 62 flows into the separation dewaterer 93 through the upstream discharge pipe 65a. As an example, the separation fluid flows into the adsorption filter 93a.

The adsorption filter 93a captures the organic solvent in the separation fluid. For this reason, the amount of the organic solvent captured by the adsorption filter 93a during the circulation by the dewatering circulator 60 increases over time. The separation fluid (mainly the water and the water vapor) that passes through the adsorption filter 93a flows into the condenser 961. The condenser 961 condenses the vapor into the separation liquid. The separation liquid that passes through the condenser 961 is discharged to the outside.

In addition, while causing the dewatering circulator 60 to stop the circulation, the controller 6 closes the first discharge valve 661 and the second discharge valve 662, opens the return valve 95, and operates the heater 93b. The heater 93b heats the adsorption filter 93a to release the organic solvent (vapor) from the adsorption filter 93a. The organic solvent flows into the condenser 962 on the return pipe 94. The condenser 962 condenses the vapor of the organic solvent into the liquid organic solvent. The condenser 962 may condense most or all of the vapor of the organic solvent. The second concentrated liquid that passes through the condenser 962 is supplied to the purification tank Tk2.

As described above, in the fourth embodiment, the separation liquid processing part 90 separates part or all of the organic solvent in the separation fluid from the separation fluid. For this reason, also in the fourth embodiment, the organic solvent in the separation fluid can be reduced. Consequently, the waste liquid processing part of the discharge destination easily processes the separation liquid. On the other hand, the separated organic solvent (second concentrated liquid) is joined in the first concentrated liquid. In the above-described example, the second concentrated liquid is supplied to the purification tank Tk2. For this reason, the discharge amount of the organic solvent can be further reduced. The second concentrated liquid may be supplied to the collection tank Tkl.

### <Second example>

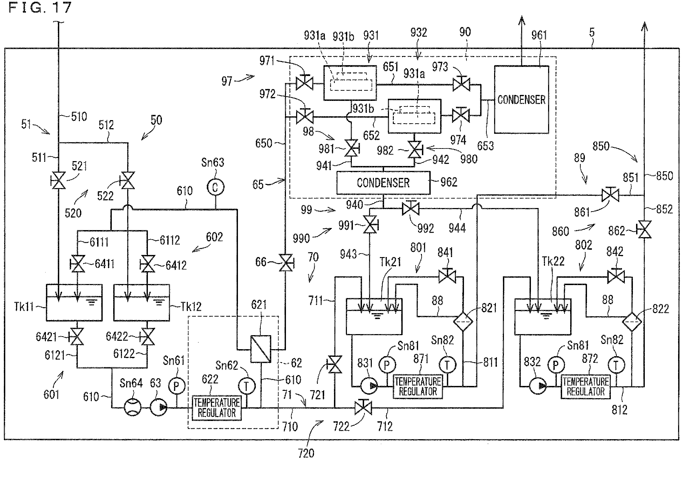

FIG. 17 is a view schematically illustrating a second example of the organic solvent collection part 5 according to the fourth embodiment. The organic solvent collection part 5 according to the second example of the fourth embodiment is different from the organic solvent collection part 5 according to the second embodiment in the existence of the separation liquid processing part 90.

In the example of FIG. 17, the separation liquid processing part 90 includes a first separation dewaterer 931, a second separation dewaterer 932, a dewatering selector 97, and a purification selector 99.

The dewatering selector 97 selects the discharge path of the separation fluid from the circulation dewaterer 62 between a path passing through the first separation dewaterer 931 and a path passing through the second separation dewaterer 932. As an example, the dewatering selector 97 includes the separation discharge pipe 65, a first branch valve 971, and a second branch valve 972. The separation discharge pipe 65 includes an upstream common pipe 650, a first branch pipe 651, a second branch pipe 652, and a downstream common pipe 653.

The upstream end of the upstream common pipe 650 is connected to the circulation dewaterer 62, and the downstream end of the upstream common pipe 650 is connected to the upstream end of the first branch pipe 651 and the upstream end of the second branch pipe 652. The downstream end of the first branch pipe 651 and the downstream end of the second branch pipe 652 are connected to the upstream end of the downstream common pipe 653. The downstream end of the downstream common pipe 653 is connected to the outside (for example, the discharge processing part of the factory equipment).

The first separation dewaterer 931 is interposed in the first branch pipe 651, and the second separation dewaterer 932 is interposed in the second branch pipe 652. An example of the configurations of the first separation dewaterer 931 and the second separation dewaterer 932 is the same as that of the separation dewaterer 93. However, in the second example, the condenser 961 is shared by the first separation dewaterer 931 and the second separation dewaterer 932. That is, the first separation dewaterer 931 includes the first adsorption filter 931a, the first heater 931b, and the condenser 961, and the second separation dewaterer 932 includes the second adsorption filter 932a, the second heater 932b, and the condenser 961. The first adsorption filter 931a and the second adsorption filter 932a are similar to the adsorption filter 93a, and the first heater 931b and the second heater 932b are similar to the heater 93b. The condenser 962 condenses the vapor of the organic solvent from each of the first adsorption filter 931a and the second adsorption filter 932a.

The first branch valve 971 is interposed in the first branch pipe 651, and the second branch valve 972 is interposed in the second branch pipe 652. The first branch valve 971 is provided at the position on the upstream side of the first separation dewaterer 931, and the second branch valve 972 is provided at the position on the upstream side of the second separation dewaterer 932. In the example of FIG. 17, a third branch valve 973 is interposed in a portion of the first branch pipe 651 on the downstream side of the first separation dewaterer 931, and a fourth branch valve 974 is interposed in a portion of the second branch pipe 652 on the downstream side of the second separation dewaterer 932.

As described later, the dewatering selector 97 can select the discharge path according to the dewatering circulation path during the circulation in the dewatering circulator 60. For example, the dewatering selector 97 selects the discharge path including the first separation dewaterer 931 during the circulation through the first dewatering circulation path 601. Thus, the separation fluid separated from the first dewatering circulation path 601 flows through the first separation dewaterer 931, and the organic solvent is adsorbed to the first adsorption filter 931a. On the other hand, the dewatering selector 97 selects the discharge path including the second separation dewaterer 932 during the circulation through the second dewatering circulation path 602. Thus, the organic solvent is adsorbed to the second adsorption filter 932a during the circulation through the second dewatering circulation path 602. Because the first dewatering circulation path 601 and the second dewatering circulation path 602 are alternately selected, the discharge path is also alternately selected.

In the example of FIG. 17, the separation liquid processing part 90 also includes the condenser 961 and a release selector 98. The condenser 961 is interposed in the downstream common pipe 653, and condenses the vapor of the separation fluid from each of the first separation dewaterer 931 and the second separation dewaterer 932.

The release selector 98 selects the supply source that supplies the organic solvent to the condenser 962 between the first adsorption filter 931a and the second adsorption filter 932a. As an example, the release selector 98 includes a common return pipe 940, a first branch pipe 941, a second branch pipe 942, and a selecting valve part 980. The upstream end of the first branch pipe 941 is connected to the first adsorption filter 931a, the upstream end of the second branch pipe 942 is connected to the second adsorption filter 932a, and the downstream end of the first branch pipe 941 and the downstream end of the second branch pipe 942 are connected to the upstream end of the common return pipe 940.

The selecting valve part 980 selects between the state in which the first adsorption filter 931a communicates with the condenser 962 and the state in which the second adsorption filter 932a communicates with the condenser 962. In the example of FIG. 17, the selecting valve part 980 includes a first release valve 981 and a second release valve 982. The first release valve 981 is interposed in the first branch pipe 941, and the second release valve 982 is interposed in the second branch pipe 942. The condenser 962 is interposed in the common return pipe 940. The condenser 962 condenses the vapor of the organic solvent and causes the second concentrated liquid to flow to the downstream side.

As described later, the release selector 98 alternately selects the supply source that supplies the second concentrated liquid between the first separation dewaterer 931 and the second separation dewaterer 932.

In the example of FIG. 17, the purification selector 99 is provided in the organic solvent collection part 5. The purification selector 99 selects the supply destination of the second concentrated liquid from the separation liquid processing part 90 between the first purification tank Tk21 and the second purification tank Tk22. As an example, the purification selector 99 includes the common return pipe 940, a first branch pipe 943, a second branch pipe 944, and a selecting valve part 990. The upstream end of the first branch pipe 943 and the upstream end of the second branch pipe 944 are connected to the downstream end of the common return pipe 940. The downstream end of the first branch pipe 943 is connected to the first purification tank Tk21, and the downstream end of the second branch pipe 944 is connected to the second purification tank Tk22.

The selecting valve part 990 selects between the state in which the condenser 962 communicates with the first purification tank Tk21 and the state in which the condenser 962 communicates with the second purification tank Tk22. In the example of FIG. 17, the selecting valve part 990 includes a first purification valve 991 and a second purification valve 992. The first purification valve 991 is interposed in the first branch pipe 943, and the second purification valve 984 is interposed in the second branch pipe 944.

As described later, the purification selector 99 alternately selects the purification tank Tk2 that supplies the second concentrated liquid between the first purification tank Tk21 and the second purification tank Tk22.

### <Example of operation of organic solvent collection part 5>

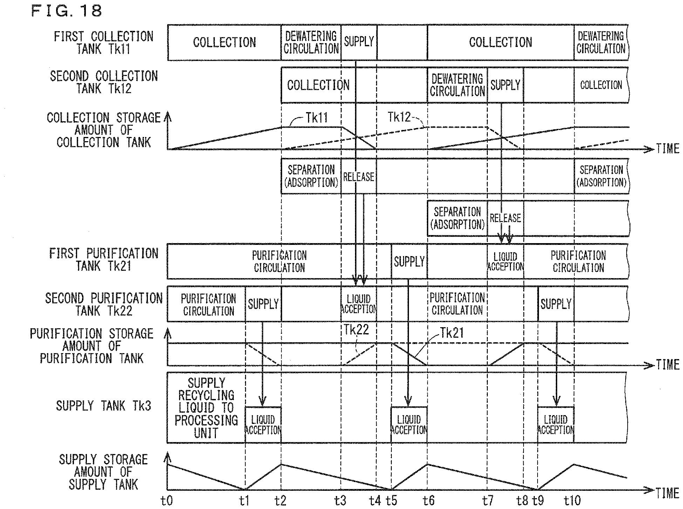

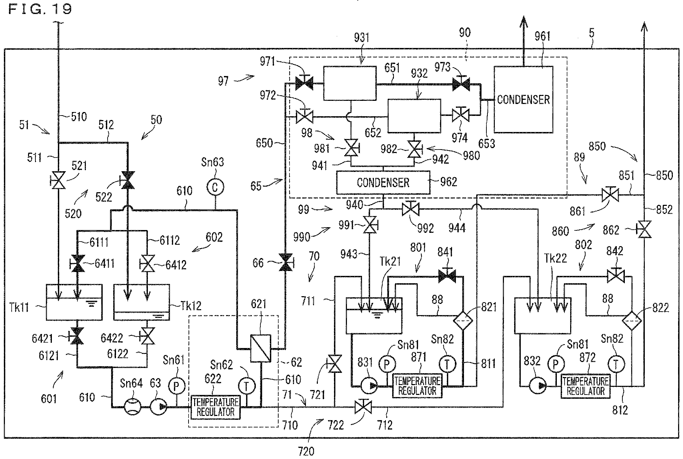

FIG. 18 is a view schematically illustrating an example of the operation of the organic solvent collection part 5 according to the second example of the fourth embodiment. FIGS. 19 to 22 are views illustrating an operation example of the organic solvent collection part 5. At this point, the operations of the collection selector 50, the dewatering circulator 60, the concentrated liquid supply part 70, the purification circulator 80, and the recycling liquid supply part 89 are the same as those in the second embodiment. For this reason, the following description focuses on the separation liquid processing part 90.

As illustrated in FIG. 18, the first separation dewaterer 931 separates the organic solvent from the separation fluid during the circulation of the mixed liquid through the first dewatering circulation path 601 including the first collection tank Tk11 (from the time point t2 to the time point t3). Specifically, as illustrated in FIG. 19, the controller 6 opens the discharge valve 66, the first branch valve 971, and the third branch valve 973, and causes the dewatering circulator 60 to start the circulation in the state in which the condenser 961 is operated. Thus, the separation fluid from the circulation dewaterer 62 passes through the first separation dewaterer 931 and the condenser 961 in this order and is discharged to the outside. Because the separation fluid flows into the first separation dewaterer 931, the amount of the organic solvent captured by the first adsorption filter 931a increases over time.

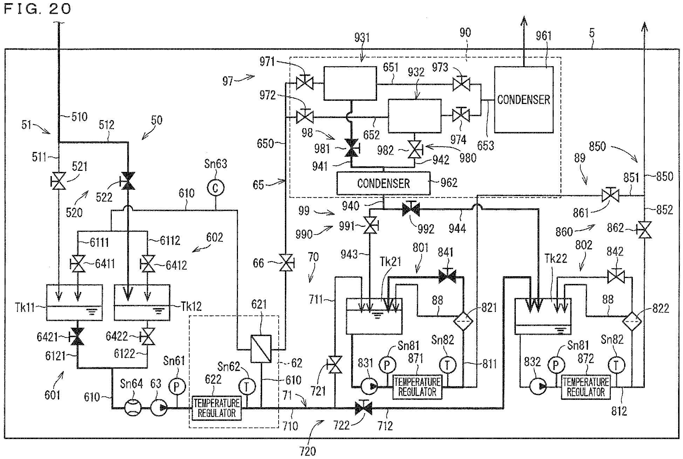

In the example of FIG. 18, the controller 6 stops the circulation through the first dewatering circulation path 601 and ends the separation of the organic solvent from the separation fluid by the first separation dewaterer 931 (time point t3). Specifically, the controller 6 closes the discharge valve 66, the first branch valve 971, and the third branch valve 973 to stop the condenser 961. Subsequently, the separation liquid processing part 90 supplies the second concentrated liquid from the first separation dewaterer 931 to the second purification tank Tk22. Specifically, as illustrated in FIG. 20, the controller 6 opens the first release valve 981 and the second purification valve 992 to operate the first heater 931b of the first separation dewaterer 931 and the condenser 962. Thus, the organic solvent is released from the first adsorption filter 931a of the first separation dewaterer 931, and supplied as the second concentrated liquid to the second purification tank Tk22 through the condenser 962.

In the example of FIG. 18, the supply of the second concentrated liquid from the first separation dewaterer 931 to the second purification tank Tk22 is performed in parallel with the supply of the first concentrated liquid from the first collection tank Tk1 1 to the second purification tank Tk22 by the concentrated liquid supply part 70 (the time point t3 to the time point t4). In the example of FIG. 18, although the supply of the second concentrated liquid from the first separation dewaterer 931 to the second purification tank Tk22 is ended at the time point t4, the present embodiment is not necessarily limited thereto. This is because the time required for releasing the organic solvent in the first adsorption filter 931a can be different from the supply time of the first concentrated liquid from the first collection tank Tk11 to the second purification tank Tk22.

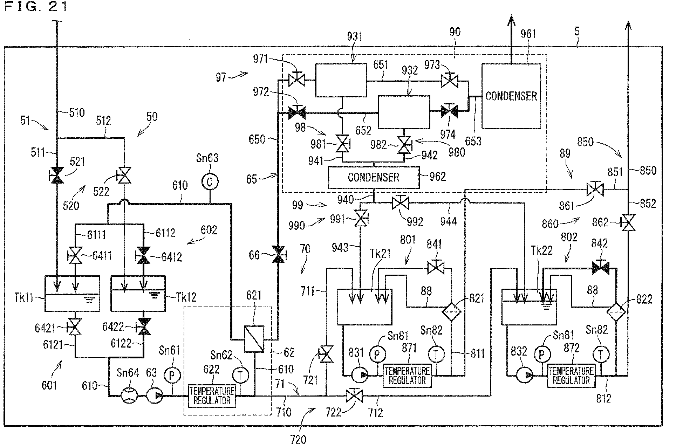

As illustrated in FIG. 18, the second separation dewaterer 932 separates the organic solvent from the separation fluid during the circulation of the mixed liquid through the second dewatering circulation path 602 including the second collection tank Tk12 (from the time point t6 to the time point t7). Specifically, as illustrated in FIG. 21, the controller 6 opens the discharge valve 66, the second branch valve 972, and the fourth branch valve 974 to operate the condenser 961. Thus, the separation fluid from the circulation dewaterer 62 passes through the second separation dewaterer 932 and the condenser 961 in this order and is discharged to the outside. Because the separation fluid flows into the second separation dewaterer 932, the amount of the organic solvent captured by the second adsorption filter 932a increases over time.

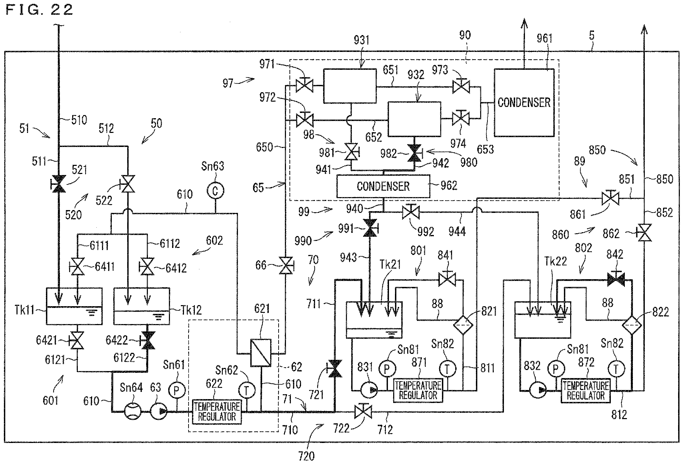

In the example of FIG. 18, the controller 6 stops the circulation through the second dewatering circulation path 602 and ends the separation of the organic solvent from the separation fluid by the second separation dewaterer 932 (the time point t7). Specifically, the controller 6 closes the discharge valve 66, the second branch valve 972, and the fourth branch valve 974 to stop the condenser 961. Subsequently, the separation liquid processing part 90 supplies the second concentrated liquid from the second separation dewaterer 932 to the first purification tank Tk21. Specifically, as illustrated in FIG. 22, the controller 6 opens the second release valve 982 and the first purification valve 991 to operate the second heater 932b of the second separation dewaterer 932 and the condenser 962. Thus, the organic solvent is released from the second adsorption filter 932a of the second separation dewaterer 932, and supplied as the second concentrated liquid to the first purification tank Tk21 through the condenser 962.

In the example of FIG. 18, the supply of the second concentrated liquid from the second separation dewaterer 932 to the first purification tank Tk21 is performed in parallel with the supply of the first concentrated liquid from the second collection tank Tk12 to the first purification tank Tk21 by the concentrated liquid supply part 70 (the time point t7 to the time point t8). In the example of FIG. 18, although the supply of the second concentrated liquid from the second separation dewaterer 932 to the first purification tank Tk21 is ended at the time point t8, the present embodiment is not necessarily limited thereto. This is because the time required for releasing the organic solvent in the second adsorption filter 932a can be different from the supply time of the first concentrated liquid from the second collection tank Tk12 to the first purification tank Tk21.

As described above, the separation liquid processing part 90 can capture the organic solvent in the separation fluid by the first adsorption filter 931a of the first separation dewaterer 931 during the circulation through the first dewatering circulation path 601, and can capture the organic solvent in the separation fluid by the second adsorption filter 932a of the second separation dewaterer 932 during the circulation through the second dewatering circulation path 602. Then, the separation liquid processing part 90 releases the organic solvent from the first adsorption filter 931a of the first separation dewaterer 931 to supply the second concentrated liquid to the second purification tank Tk22 while the circulation through the first dewatering circulation path 601 is stopped, and releases the organic solvent from the second adsorption filter 932a of the second separation dewaterer 932 to supply the second concentrated liquid to the second purification tank Tk22 while the circulation through the second dewatering circulation path 602 is stopped.

Moreover, the two first separation dewaterers 931 and second separation dewaterers 932 are provided as the separation dewaterers 93, so that one of them can release the organic solvent while the other captures the organic solvent. For example, in FIG. 18, the release from the first adsorption filter 931a of the first separation dewaterer 931 may be ended after the time point t5. In this case, the release from the first adsorption filter 931a of the first separation dewaterer 931 is performed in parallel with the adsorption of the second adsorption filter 932a of the second separation dewaterer 932. The release time can be lengthened, so that the organic solvent captured in the first adsorption filter 931a can be more reliably released. The same applies to the second adsorption filter 932a.

As described above, the organic solvent collection apparatus (organic solvent collection part 5), the substrate processing apparatus 100, and the organic solvent collection method have been described in detail, but the above description is an example in all aspects, and this disclosure is not limited thereto. In addition, the various modifications described above can be applied in combination as long as they do not contradict each other. Many modifications not illustrated can be envisaged without departing from the scope of the present disclosure.

The present disclosure includes the following aspects.

A first aspect is an organic solvent collection apparatus includes: at least one collection tank that stores a mixed liquid of an organic solvent and water discharged from a processing unit that processes a substrate; a dewatering circulator that includes at least one dewatering circulation pipe connected to the at least one collection tank and a circulation dewaterer provided in the at least one dewatering circulation pipe and separating water from the mixed liquid, the dewatering circulator generating a first concentrated liquid that is the mixed liquid in which a solvent concentration of the organic solvent is increased by circulation through the at least one dewatering circulation pipe; and a purification circulator that includes at least one purification circulation pipe circulating the first concentrated liquid and at least one filter provided in the at least one purification circulation pipe and capturing an impurity of the first concentrated liquid, the purification circulator circulating the first concentrated liquid through the at least one purification circulation pipe to generate a recycling liquid that is the first concentrated liquid in which a content rate of the impurity is reduced.

A second aspect is the organic solvent collection apparatus according to the first aspect, in which the circulation dewaterer includes a membrane separator having a separation membrane that allows water to pass therethrough.

A third aspect is the organic solvent collection apparatus according to the first or second aspect, in which the circulation dewaterer includes a dewatering temperature regulator that heats the mixed liquid.

A fourth aspect is the organic solvent collection apparatus according to the first or second aspect, in which the purification circulator further includes a purification temperature regulator that heats or cools the first concentrated liquid flowing through the at least one purification circulation pipe.

A fifth aspect is the organic solvent collection apparatus according to the fourth aspect, in which the purification temperature regulator cools the first concentrated liquid flowing through the at least one purification circulation pipe.

A sixth aspect is the organic solvent collection apparatus according to any one of the first to fifth aspects, further includes: at least one purification tank; and a first liquid sending pipe that causes the first concentrated liquid from the at least one collection tank to flow toward the at least one purification tank, in which the at least one purification circulation pipe returns the first concentrated liquid from the at least one purification tank to the at least one purification tank.

A seventh aspect is the organic solvent collection apparatus according to any one of the first to sixth aspects, further includes a collection selector that selects a collection destination of the mixed liquid from the processing unit between a first collection tank and a second collection tank included in the at least one collection tank, in which the at least one dewatering circulation pipe includes a first dewatering circulation pipe and a second dewatering circulation pipe, the first dewatering circulation pipe returns the mixed liquid from the first collection tank to the first collection tank, and the second dewatering circulation pipe returns the mixed liquid from the second collection tank to the second collection tank.

An eighth aspect is the organic solvent collection apparatus according to the seventh aspect, in which the first dewatering circulation pipe includes a first upstream pipe having an upstream end connected to the first collection tank, a first downstream pipe having a downstream end connected to the first collection tank, and a common circulation pipe connected between the first upstream pipe and the first downstream pipe, the second dewatering circulation pipe includes a second upstream pipe having an upstream end connected to the second collection tank, a second downstream pipe having a downstream end connected to the second collection tank, and the common circulation pipe connected between the second upstream pipe and the second downstream pipe, the circulation dewaterer is provided in the common circulation pipe, and the dewatering circulator further includes a circulation selecting valve part that selects between circulation through the first dewatering circulation pipe and circulation through the second dewatering circulation pipe.

A ninth aspect is the organic solvent collection apparatus according to any one of the first to eighth aspects, further includes a concentrated liquid supply part that selects a supply destination from the at least one collection tank between a first purification tank and a second purification tank included in the at least one purification tank, in which the at least one purification circulation pipe includes a first purification circulation pipe and a second purification circulation pipe, the first purification circulation pipe returns the first concentrated liquid from the first purification tank to the first purification tank, the second purification circulation pipe returns the first concentrated liquid from the second purification tank to the second purification tank, the at least one filter includes a first filter and a second filter, the first filter is provided in the first purification circulation pipe, and the second filter is provided in the second purification circulation pipe.

A tenth aspect is the organic solvent collection apparatus according to any one of the first to ninth aspects, includes: a separation discharge pipe through which a separation fluid containing water separated from the mixed liquid by the circulation dewaterer as a main component flows; and a decomposer that is provided in the separation discharge pipe and decomposes the organic solvent contained in the separation fluid.

An eleventh aspect is the organic solvent collection apparatus according to the tenth aspect, further includes a condenser that is provided in the separation discharge pipe on an upstream side of the decomposer and condenses vapor of the organic solvent contained in the separation fluid, in which the decomposer electrolyzes the organic solvent of liquid.

A twelfth aspect is the organic solvent collection apparatus according to any one of the first to tenth aspects, further includes: a separation discharge pipe through which a separation fluid containing water as a main component separated from the mixed liquid by the circulation dewaterer flows; a separation dewaterer that is provided in the separation discharge pipe and separates the organic solvent from the separation fluid; and a return pipe that is connected to the separation dewaterer and joins a second concentrated liquid containing the organic solvent separated from the separation fluid by the separation dewaterer in the first concentrated liquid.

A thirteenth aspect is the organic solvent collection apparatus according to the twelfth aspect, in which the separation dewaterer includes: at least one adsorption filter that is provided in the separation discharge pipe and adsorbs the organic solvent; at least one heater that heats the at least one adsorption filter to release the organic solvent from the at least one adsorption filter and causes the organic solvent to flow out to the return pipe; and a condenser provided in the return pipe.

A fourteenth aspect is the organic solvent collection apparatus according to the thirteenth aspect, includes: a dewatering selector that selects the at least one adsorption filter through which the separation fluid flows between a first adsorption filter and a second adsorption filter in included the at least adsorption filter; and a release selector that selects the adsorption filter communicating with the condenser between the first adsorption filter and the second adsorption filter, in which the at least one heater includes a first heater and a second heater, the first heater heats the first adsorption filter, the second heater heats the second adsorption filter.

A fifteenth aspect is a substrate processing apparatus, includes: the organic solvent collection apparatus according to any one of the first to fourteenth aspects; the processing unit; a supply tank to which the recycling liquid is supplied and that stores the recycling liquid; and a second liquid sending pipe that connects the supply tank and the processing unit.

A sixteenth aspect is an organic solvent collection method including: a dewatering circulation step of circulating a mixed liquid of an organic solvent and water through a dewatering circulation path including a circulation dewaterer that separates water from the mixed liquid to generate a first concentrated liquid in which a solvent concentration of the organic solvent in the mixed liquid is increased; and a purification circulation step of circulating the first concentrated liquid through a purification circulation path including a filter that captures an impurity in the first concentrated liquid to generate a recycling liquid in which a content rate of the impurity in the first concentrated liquid is reduced.

A seventeenth aspect is the organic solvent collection method according to the sixteenth aspect, in which the mixed liquid is heated in the dewatering circulation step and the first concentrated liquid is cooled in the purification circulation step.

According to the first, fifteenth, and sixteenth aspects, the mixed liquid can be circulated through the dewatering circulation pipe under a condition corresponding to the circulation dewaterer, and the first concentrated liquid can be circulated through the purification circulation pipe under a condition corresponding to the filter.

According to the second aspect, the apparatus scale can be reduced as compared with the distiller.

According to the third aspect, the water can be efficiently separated from the mixed liquid.

According to the fourth aspect, the temperature of the mixed liquid can be adjusted to the temperature suitable for the filter.

According to the fifth aspect, even when the purification temperature regulator increases the temperature of the mixed liquid, the high-temperature first concentrated liquid can be cooled to near the normal temperature. For this reason, thermal deformation of the filter can be reduced, and the filter can capture the impurities with high efficiency.

According to the sixth aspect, when the first concentrated liquid having the high temperature is supplied to the purification tank through the first liquid sending pipe, the temperature of the first concentrated liquid can be reduced by heat dissipation during passage through the first liquid sending pipe.

According to the seventh aspect, the mixed liquid from the processing unit can be collected in the second collection tank during the circulation of the mixed liquid through the first dewatering circulation pipe, and the mixed liquid from the processing unit can be collected in the first collection tank during the circulation of the mixed liquid through the second dewatering circulation pipe. In short, the mixed liquid from the processing unit can be collected even during the circulation of the mixed liquid through the first dewatering circulation pipe or during the circulation of the mixed liquid through the second dewatering circulation pipe. Conversely, the mixed liquid having the low solvent concentration discharged from the processing unit can hardly flow into the mixed liquid during the circulation. For this reason, the drop in the solvent concentration of the mixed liquid in the circulation can be reduced.

According to the eighth aspect, the circulation dewaterer is commonly used in the first dewatering circulation pipe and the second dewatering circulation pipe, so that the apparatus scale and the manufacturing cost can be reduced.

According to the ninth aspect, the circulation through the first filter and the circulation through the second filter can be performed in parallel. For this reason, the recycling liquid having the lower impurity content rate can be generated.

According to the tenth aspect, the liquid discharge process of the separation fluid at the discharge destination can be simplified.

According to the eleventh aspect, the organic solvent is electrolyzed after the vapor of the organic solvent is condensed, so that the organic solvent can be more reliably decomposed.

According to the twelfth aspect, the second concentrated liquid containing the organic solvent in the separation fluid separated by the circulation dewaterer joins in the first concentrated liquid. For this reason, the discharge amount of the organic solvent can be further reduced.

According to the thirteenth aspect, the organic solvent can be more reliably separated from the separation fluid having the low solvent concentration.

According to the fourteenth aspect, the release by one of the first and second adsorption filters can be performed in parallel with the adsorption of the other of the first and second adsorption filters.

According to the seventeenth aspect, the high-temperature first concentrated liquid can be cooled to near normal temperature. For this reason, thermal deformation of the filter can be reduced, and the filter can capture the impurities with high efficiency.

While the disclosure has been shown and described in detail, the foregoing description is in all aspects illustrative and not restrictive. It is therefore understood that numerous modifications and variations can be devised.

## Claims

1. An organic solvent collection apparatus comprising:
at least one collection tank (Tk1, Tk 11, Tk12) that stores a mixed liquid of an organic solvent and water discharged from a processing unit (4) processing a substrate (W);
a dewatering circulator (60) that includes at least one dewatering circulation pipe (61, 611, 612) connected to said at least one collection tank (Tk1, Tk11, Tk12) and a circulation dewaterer (62) provided in said at least one dewatering circulation pipe (61, 611, 612) and separating water from said mixed liquid, said dewatering circulator (60) generating a first concentrated liquid that is said mixed liquid in which a solvent concentration of said organic solvent is increased by circulation through said at least one dewatering circulation pipe (61, 611, 612); and
a purification circulator (80) that includes at least one purification circulation pipe (81, 811, 812) circulating said first concentrated liquid and at least one filter (82, 821, 822) provided in said at least one purification circulation pipe (81, 811, 812) and capturing an impurity of said first concentrated liquid, said purification circulator (80) circulating said first concentrated liquid through said at least one purification circulation pipe (81, 811, 812) to generate a recycling liquid that is said first concentrated liquid in which a content rate of said impurity is reduced.

2. The organic solvent collection apparatus according to claim 1, wherein
said circulation dewaterer (62) includes a membrane separator (621) including a separation membrane that allows water to pass therethrough.

3. The organic solvent collection apparatus according to claim 1 or 2, wherein said circulation dewaterer (62) includes a dewatering temperature regulator (622) that heats said mixed solution.

4. The organic solvent collection apparatus according to claim 1 or 2, wherein
said purification circulator (80) further includes a purification temperature regulator (87, 871, 872) that heats or cools said first concentrated liquid flowing through said at least one purification circulation pipe (81, 811, 812).

5. The organic solvent collection apparatus according to claim 4, wherein
said purification temperature regulator (87, 871, 872) cools said first concentrated liquid flowing through said at least one purification circulation pipe (81, 811, 812).

6. The organic solvent collection apparatus according to any one of claims 1 to 5, further comprising:
at least one purification tank (Tk2, Tk21, Tk22); and
a first liquid sending pipe (71) that causes said first concentrated liquid from said at least one collection tank (Tk1, Tk11, Tk12) to flow toward said at least one purification tank (Tk2, Tk21, Tk22), wherein
said at least one purification circulation pipe (81, 811, 812) returns said first concentrated liquid from said at least one purification tank (Tk2, Tk21, Tk22) to said at least one purification tank (Tk2, Tk21, Tk22).

7. The organic solvent collection apparatus according to any one of claims 1 to 6, further comprising
a collection selector (50) that selects a collection destination of said mixed liquid from said processing unit (4) between a first collection tank (Tk11) and a second collection tank (Tk12) included in said at least one collection tank, wherein
said at least one dewatering circulation pipe includes a first dewatering circulation pipe (611) and a second dewatering circulation pipe (612),
said first dewatering circulation pipe (611) returns said mixed liquid from said first collection tank (Tk11) to said first collection tank (Tk11), and
said second dewatering circulation pipe (612) returns said mixed liquid from said second collection tank (Tk 12) to said second collection tank (Tk12).

8. The organic solvent collection apparatus according to claim 7, wherein
said first dewatering circulation pipe (611) includes:
a first upstream pipe (6121) having an upstream end connected to said first collection tank (Tk11),
a first downstream pipe (6111) having a downstream end connected to said first collection tank (Tk11), and
a common circulation pipe (610) connected between said first upstream pipe (6121) and said first downstream pipe (6111),
said second dewatering circulation pipe (612) includes:
a second upstream pipe (6122) having an upstream end connected to said second collection tank (Tk12);
a second downstream pipe (6112) having a downstream end connected to said second collection tank (Tk12); and
said common circulation pipe (610) connected between said second upstream pipe (6122) and said second downstream pipe (6112),
said circulation dewaterer (62) is provided in said common circulation pipe (610), and
said dewatering circulator (60) further includes a circulation selecting valve part (640) that selects between circulation through said first dewatering circulation pipe (611) and circulation through said second dewatering circulation pipe (612).

9. The organic solvent collection apparatus according to claim 6, further comprising
a concentrated liquid supply part (70) that selects a supply destination from said at least one collection tank (Tk1, Tk11, Tk12) between a first purification tank (Tk21) and a second purification tank (Tk22) included in said at least one purification tank, wherein
said at least one purification circulation pipe includes a first purification circulation pipe (811) and a second purification circulation pipe (812),
said first purification circulation pipe (811) returns said first concentrated liquid from said first purification tank (Tk21) to said first purification tank (Tk21),
said second purification circulation pipe (812) returns said first concentrated liquid from said second purification tank (Tk22) to said second purification tank (Tk22),
said at least one filter includes a first filter (821) and a second filter (822),
said first filter (821) is provided in said first purification circulation pipe (811), and
said second filter (822) is provided in said second purification circulation pipe (812).

10. The organic solvent collection apparatus according to any one of claims 1 to 9, comprising:
a separation discharge pipe (65) through which a separation fluid containing water separated from said mixed liquid by said circulation dewaterer (62) as a main component flows; and
a decomposer (92) that is provided in said separation discharge pipe (65) and decomposes said organic solvent contained in said separation fluid.

11. The organic solvent collection apparatus according to claim 10, further comprising
a condenser (91, 962) that is provided in said separation discharge pipe (65) on an upstream side of said decomposer (92) and condenses vapor of said organic solvent contained in said separation fluid, wherein
said decomposer (92) electrolyzes said organic solvent of liquid.

12. The organic solvent collection apparatus according to any one of claims 1 to 10, further comprising:
a separation discharge pipe (65) through which a separation fluid containing water as a main component separated from said mixed liquid by said circulation dewaterer (62) flows;
a separation dewaterer (93) that is provided in said separation discharge pipe (65) and separates said organic solvent from said separation fluid; and
a return pipe (94) that is connected to said separation dewaterer (93) and joins a second concentrated liquid containing said organic solvent separated from said separation fluid by said separation dewaterer (93) in said first concentrated liquid.

13. The organic solvent collection apparatus according to claim 12, wherein
said separation dewaterer (93) includes:
at least one adsorption filter (93a, 931a, 932a) that is provided in said separation discharge pipe (65) and adsorbs said organic solvent;
at least one heater (93b, 931b, 932b) that heats said at least one adsorption filter (93a, 931a, 932a) to release said organic solvent from said at least one adsorption filter (93a, 931a, 932a) and causes said organic solvent to flow out to said return pipe (94); and
a condenser (91, 962) provided in said return pipe (94).

14. The organic solvent collection apparatus according to claim 13, comprising:
a dewatering selector (97) that selects said at least one adsorption filter through which said separation fluid flows between a first adsorption filter (931a) and a second adsorption filter (932a) in included said at least adsorption filter; and
a release selector (98) that selects said adsorption filter communicating with said condenser (91, 962) between said first adsorption filter (931a) and said second adsorption filter (932a), wherein
said at least one heater includes a first heater (931b) and a second heater (932b),
said first heater (931b) heats said first adsorption filter (93 1a), and
said second heater (932b) heats said second adsorption filter (932a).

15. A substrate processing apparatus, comprising:
the organic solvent collection apparatus according to any one of claims 1 to 14;
said processing unit (4);
a supply tank (Tk3) to which said recycling liquid is supplied and that stores said recycling liquid; and
a second liquid sending pipe (85) that connects said supply tank (Tk3) and said processing unit (4).

16. An organic solvent collection method comprising:
a dewatering circulation step (S1) of circulating a mixed liquid of an organic solvent and water through a dewatering circulation path (601, 602) including a circulation dewaterer (62) that separates water from the mixed liquid to generate a first concentrated liquid in which a solvent concentration of the organic solvent in the mixed liquid is increased; and
a purification circulation step (S3) of circulating the first concentrated liquid through a purification circulation path (801, 802) including a filter (82, 821, 822) that captures an impurity in the first concentrated liquid to generate a recycling liquid in which a content rate of the impurity in the first concentrated liquid is reduced.

17. The organic solvent collection method according to claim 16, wherein
said mixed liquid is heated in said dewatering circulation step (S1) and said first concentrated liquid is cooled in said purification circulation step (S3).
